# EUROPEAN PATENT APPLICATION

(11) **EP 4 119 946 A1**
(43) Date of publication of application: **18.01.2023**
(21) Application number: 21767268.2
(22) Date of filing: 12.03.2021
(51) Int. Cl.: G01N 33/543, G01N 33/68, G01N 33/569

(54) **METHOD AND DEVICE FOR SCREENING ANTIGEN EPITOPE POLYPEPTIDE**

(30) Priority: 13.03.2020 CN 202010176984; 14.04.2020 CN 202010291238; 30.12.2020 CN 202011629071
(71) Applicant: Icarbonx (Zhuhai) Company Limited, Zhuhai, Guangdong 519000 (CN)
(72) Inventor: LI, Yingrui, Zhuhai, Guangdong 519000 (CN); WANG, Jian, Zhuhai, Guangdong 519000 (CN); ZHENG, Hancheng, Zhuhai, Guangdong 519000 (CN); LIU, Binghang, Zhuhai, Guangdong 519000 (CN); YAN, Mingchen, Zhuhai, Guangdong 519000 (CN); CAO, Jiamin, Zhuhai, Guangdong 519000 (CN); LI, Zhenyu, Zhuhai, Guangdong 519000 (CN); SHEN, Linghao, Zhuhai, Guangdong 519000 (CN); LI, Danni, Zhuhai, Guangdong 519000 (CN); DING, Qiuxia, Zhuhai, Guangdong 519000 (CN); SUN, Jing, Zhuhai, Guangdong 519000 (CN); LIANG, Zhiying, Zhuhai, Guangdong 519000 (CN); WANG, Jun, Zhuhai, Guangdong 519000 (CN)
(74) Representative: Barker Brettell LLP
(86) International application number: PCT/CN2021/080636
(87) International publication number: WO 2021/180232

(57) **Abstract**

Provided is a method and a device for screening an antigen epitope polypeptide. The screening method includes: predicting one or more antigen epitopes with all proteome sequences of a target coronavirus to obtain a predicted epitope region; screening a polypeptide with a differential response to a positive serum sample infected by the target coronavirus and a control serum sample with a polypeptide chip technology, and recording the polypeptide as a differential peptide fragment; aligning the differential peptide fragment with all proteome sequences of the target coronavirus to obtain a first conserved motif region; screening regions meeting epitope screening conditions from the predicted epitope region and the first conserved motif region to obtain the antigen epitope, wherein the epitope screening conditions comprise a non-phosphorylation region and/or an extracellular region of the target coronavirus. A batch of polypeptides related to coronavirus infection specificity are screened and obtained by using polypeptide chip technology with low cost and high flux, and a more powerful polypeptide product is provided for the prevention and control of this type of coronavirus.

## Description

### Technical Field

The present invention relates to the field of immunology, and specifically, to a method and device for screening an antigen epitope polypeptide.

### Background

Currently, Corona Virus Disease 2019 (COVID-19) caused by Severe Acute Respiratory Syndrome Coronavirus 2 (SARS-CoV-2) infection is wreaking havoc around the world. As of December 11, 2020, globally, there have been 70,714,214 SARS-CoV-2 infections, including 1,588,277 deaths. As the epidemic situation develops rapidly, and no effective drug has yet been found, a specific coronavirus vaccine for infection prevention is the hope of reducing infections and curbing the worsening of the epidemic situation.

A convention vaccine includes a live attenuated vaccine, an inactivated vaccine, and the like. A virus strain is required to be used during preparation. Although the immunogenicity is high, there is the possibility of virus reversion and potential pathogenic risks, resulting in relatively low safety. In recent years, various novel vaccines including a DNA recombinant vaccine, synthetic peptide vaccine and the like have been emerged one after another. However, since a vector commonly used by the DNA recombinant vaccine is an adenovirus, a vaccinia virus, or an SV40 virus, there are still some doubts about the in vivo safety of such vectors currently, so that there is still a great need to develop a safer next-generation vaccine. The polypeptide vaccine is a vaccine that is prepared by means of a chemical synthesis method according to an amino acid sequence of certain known or predicted antigen epitope in a pathogen antigen gene. Since the polypeptide vaccine is chemically synthesized, virulence reversion or incomplete inactivation does not exist. In addition, specific antigen epitope may be selected, so that the polypeptide vaccine has become a hot research point for vaccine development today. In a plurality of fields including tumor vaccines, there have been several studies have been published, and clinical trials are underway as well.

As described above, in view of the current global pandemic of novel coronavirus pneumonia, there is an urgent need to develop corresponding vaccines, especially the polypeptide vaccine.

### Summary

The present invention is mainly intended to provide a method and device for screening an antigen epitope polypeptide, to provide a corresponding polypeptide product that is developed for the polypeptide of such a novel virus.

A first aspect of this application provides a method for screening an antigen epitope. The screening method includes: using all proteome sequences of a target coronavirus to perform antigen epitope prediction, to obtain a predicted epitope region; using a polypeptide chip technology to screen a polypeptide with a differential response to a positive serum sample infected by the target coronavirus and a control serum sample, and recording the polypeptide as a differential peptide fragment; aligning the differential peptide fragment with all proteome sequences of the target coronavirus to obtain a first conserved motif region; and screening regions meeting epitope screening conditions from the predicted epitope region and the first conserved motif region to obtain the antigen epitope. The epitope screening conditions include a non-phosphorylation region and/or an extracellular region of the target coronavirus.

Further, the operation of using all proteome sequences of the target coronavirus to perform antigen epitope prediction, to obtain the predicted epitope region includes: using all proteome sequences of the target coronavirus to perform antigen epitope prediction by means of various methods, and screening epitope with a length of 8 to 20, preferably 10 to 15 amino acids, to obtain candidate prediction epitope; screening the candidate prediction epitope according to epitope and/or hydrophilicity-hydrophobicity that HLA is able to present in a specific population, to obtain the predicted epitope region; and preferably, screening, from the candidate prediction epitope, the epitope that the HLA is able to present in a Chinese population, and/or removing, from the candidate prediction epitope, the epitope of which hydrophobicity is higher than a first hydrophobic threshold, to obtain the predicted epitope region. Preferably, the epitope of which hydrophobicity is higher than the first hydrophobic threshold refers to epitope that the proportion of hydrophobic amino acids is greater than 45% and a hydrophobicity score is greater than 3.

Further, the operation of using an immune characterization method to screen the polypeptide with the differential response to the positive serum sample infected by the target coronavirus and the control serum sample, and recording the polypeptide as the differential peptide fragment includes: selecting the positive serum sample infected by the target coronavirus, a negative control serum sample and a control serum sample of another lung disease, where the another lung disease refers to a lung disease caused by infection of a virus other than the target coronavirus; using the immune characterization method to combine the positive serum sample, the negative control serum sample and the control serum sample of the another lung disease with a polypeptide array chip, to obtain signal values responsive to combined peptide fragments; for each combined peptide fragment, calculating a p value when there is a difference between the signal value of the positive serum sample and the signal value of the negative control serum sample, recording the p value as a first p value, and simultaneously, calculating a p value when there is a difference between the signal value of the positive serum sample and the signal value of the control serum sample of the another lung disease, and recording the p value as a second p value; and retaining all combined peptide fragments of which first p values and second p values simultaneously meet a difference threshold, to obtain the differential peptide fragment. The difference threshold is preferably < 0.05.

Further, log10 conversion is performed on the signal value of the combined peptide fragment, and a conversed log value is used as a feature. By means of a single-tail T test, the p value of each feature when there is a difference between the positive serum sample and the negative control serum sample is calculated, and multiple hypothesis test correction is performed on the p value to obtain the first p value; the p value of the corresponding feature when there is a difference between the positive serum sample and the control serum sample of the another lung disease is simultaneously calculated, multiple hypothesis test correction is performed on the p value, and the p value is recorded as the second p value; and all combined peptide fragments of which first p values are less than the difference threshold and second p values are less than the difference threshold simultaneously are screened, to obtain the differential peptide fragment.

Further, the operation of aligning the differential peptide fragment with all proteome sequences of the target coronavirus to obtain the first conserved motif region includes: using a single amino acid as a unit, calculating a distribution of p1 values where the signal value of the combined peptide fragment covering the amino acid and matching the amino acid differs between the positive serum sample and the negative control serum sample and the control serum sample of the another lung disease, and simultaneously calculating a distribution of p2 values where the signal value of the combined peptide fragment covering the amino acid and not matching the amino acid differs between the positive serum sample and the negative control serum sample and the control serum sample of the another lung disease, where the distribution of p1 values is remarkably lower than the distribution of p2 values, and the amino acid is a first conserved site; and aligning the differential peptide fragment with all proteome sequences of the target coronavirus, and selecting, from matching regions, a region that has the first conserved site and has hydrophobicity lower than a second hydrophobic threshold, to obtain a first conserved motif region. Preferably, the region of which hydrophobicity is lower than the second hydrophobic threshold refers to a region that the proportion of the hydrophobic amino acids is less than or equal to 45% and the hydrophobicity score is less than or equal to 3. Preferably, the differential peptide fragment is a differential peptide fragment that is able to completely match all proteome sequences of the target coronavirus.

Further, before regions meeting the epitope screening condition are screened from the predicted epitope region and the first conserved motif region, the screening method further includes: aligning the differential peptide fragment with a protein sequence of a coronavirus family to obtain a second conserved motif region. Preferably, the operation of aligning the differential peptide fragment with a protein sequence of the coronavirus family to obtain the second conserved motif region includes: aligning the differential peptide fragment with the protein sequence of the coronavirus family, and selecting, from the matching regions, a region of which amino acid site meets the following region screening condition as the second conserved motif region. In all of the differential peptide fragments covering the amino acids, the ratio of the differential peptide fragments matching the amino acids meets a matching ratio threshold; and preferably, the matching ratio threshold is greater than or equal to 75%.

Further, the epitope screening condition in the third region screening module includes at least one of the following: (a) overlapping with the second conserved motif region; (b) a comparison score with a human proteome sequence being lower than a comparison threshold; and (c) meeting a plurality of the following performance indexes: 1) the covering number of the differential peptide fragment being ≥3; 2) hydrophilicity being within a hydrophilic threshold range; and 3) an accessibility score, a Beta turn and a multi-alignment score being all in the top 100. That the comparison score is lower than the comparison threshold means that a/b ≤ 0.8, where a is a matching score that a sequence of a region to be screened is aligned with the human proteome sequence, and b is a matching score that the sequence of the region to be screened is aligned with all proteome sequences of the target coronavirus. preferably, the operation of screening regions meeting epitope screening conditions from the predicted epitope region and the first conserved motif region to obtain the antigen epitope polypeptide includes: merging the predicted epitope region and the first conserved motif region according to one of the following merging conditions: 1) there is an inclusion relation between the two regions; and 2) the two regions are predicted as antigen epitope regions by at least two different methods, to obtain a first candidate epitope region; screening a region overlapping with the second conserved motif region from the first candidate epitope region as a second candidate epitope region; screening, from the second candidate epitope region, a region of which comparison score with the human proteome sequence is lower than the comparison threshold, as a third candidate epitope region; screening and retaining the non-phosphorylation region and/or the extracellular region in the proteome sequence of the target coronavirus from the third candidate epitope region, as a fourth candidate epitope region; comprehensively sorting the fourth candidate epitope region according to accessibility, the beta turn, the hydrophilicity, the covering number of the differential peptide fragments and a multi-alignment result, and then performing optimal selection, to obtain the antigen epitope polypeptide of the target coronavirus. More preferably, after optimal selection is performed, the screening method further includes removing a region including mutations. Preferably, the target coronavirus is SARS-CoV-2.

A fourteenth aspect of this application provides a device for screening an antigen epitope polypeptide. The screening device includes: an epitope prediction module, configured to use all proteome sequences of a target coronavirus to perform antigen epitope prediction, to obtain a predicted epitope region; a differential peptide fragment screening module, configured to use a polypeptide chip technology to screen a polypeptide with a differential response to a positive serum sample infected by the target coronavirus and a control serum sample, and record the polypeptide as a differential peptide fragment; a first region screening module, configured to align the differential peptide fragment with all proteome sequences of the target coronavirus to obtain a first conserved motif region; and a third region screening module, configured to screen regions meeting epitope screening conditions from the predicted epitope region and the first conserved motif region to obtain the antigen epitope. The epitope screening conditions include a non-phosphorylation region and/or an extracellular region of the target coronavirus.

Further, the epitope prediction module includes: a first candidate epitope screening module, configured to use all proteome sequences of the target coronavirus to perform antigen epitope prediction by means of various methods, and screen epitope with a length of 8 to 20, preferably 10 to 15 amino acids, to obtain candidate prediction epitope; and a second candidate epitope screening module, configured to screen the candidate prediction epitope according to epitope and/or hydrophilicity-hydrophobicity that HLA is able to present in a specific population, to obtain the predicted epitope region.

Further, the second candidate epitope screening module includes: a population epitope screening module, configured to screen, from the candidate prediction epitope, the epitope that the HLA is able to present in a Chinese population; and/or a hydrophobicity screening module, configured to remove, from the candidate prediction epitope, the epitope of which hydrophobicity is higher than a first hydrophobic threshold, to obtain the predicted epitope region. Preferably, the epitope of which hydrophobicity is higher than the first hydrophobic threshold refers to epitope that the proportion of hydrophobic amino acids is greater than 45% and a hydrophobicity score is greater than 3.

Further, the differential peptide fragment screening module includes a first screening module. The first screening module includes: a sample selection unit, configured to select the positive serum sample infected by the target coronavirus, a negative control serum sample and a control serum sample of another lung disease, where the another lung disease refers to a lung disease caused by infection of a virus other than the target coronavirus; a signal acquisition unit, configured to use an immune characterization method to combine the positive serum sample, the negative control serum sample and the control serum sample of the another lung disease with a polypeptide array chip, to obtain signal values responsive to combined peptide fragments; and a differential peptide fragment screening unit, configured to, for each combined peptide fragment, calculate a p value when there is a difference between the signal value of the positive serum sample and the signal value of the negative control serum sample, record the p value as a first p value, and simultaneously, calculate a p value when there is a difference between the signal value of the positive serum sample and the signal value of the control serum sample of the another lung disease, and record the p value as a second p value; and retain all combined peptide fragments of which first p values and second p values simultaneously meet a difference threshold, to obtain the differential peptide fragment. The difference threshold is preferably < 0.05.

Further, the differential peptide fragment screening unit includes: a signal conversion sub-unit, configured to perform log10 conversion on the signal value of the combined peptide fragment; and a differential peptide fragment screening sub-unit, configured to use a conversed log value as a feature, by means of a single-tail T test, calculate the p value of each feature when there is a difference between the positive serum sample and the negative control serum sample, and perform multiple hypothesis test correction on the p value to obtain the first p value; simultaneously calculate the p value of the corresponding feature when there is a difference between the positive serum sample and the control serum sample of the another lung disease, perform multiple hypothesis test correction on the p value, and record the p value as the second p value; and screen all combined peptide fragments of which first p values are less than the difference threshold and second p values are less than the difference threshold simultaneously, to obtain the differential peptide fragment.

Further, the first region screening module includes: a conserved site screening module, configured to use a single amino acid as a unit, calculate a distribution of p1 values where the signal value of the combined peptide fragment covering the amino acid and matching the amino acid differs between the positive serum sample and the negative control serum sample, simultaneously calculate a distribution of p2 values where the signal value of the combined peptide fragment covering the amino acid and not matching the amino acid differs between the positive serum sample and the negative control serum sample, and record the amino acid that the distribution of p1 values is remarkably lower than the distribution of p2 values as a first conserved site; and a first conserved motif screening module, configured to align the differential peptide fragment with all proteome sequences of the target coronavirus, and select, from matching regions, a region that has the first conserved site and has hydrophobicity lower than a second hydrophobic threshold, to obtain a first conserved motif region. Preferably, the region of which hydrophobicity is lower than the second hydrophobic threshold refers to a region that the proportion of the hydrophobic amino acids is less than or equal to 45% and the hydrophobicity score is less than or equal to 3. Preferably, the differential peptide fragment is a differential peptide fragment that is able to completely match all proteome sequences of the target coronavirus.

Further, the screening device further includes a second region screening module. Preferably, the second region screening module includes: an alignment module, configured to align the differential peptide fragment with a protein sequence of a coronavirus family; and a second conserved motif screening module, configured to select, from the matching regions, a region of which amino acid site meets the following region screening condition as the second conserved motif region. In all of the differential peptide fragments covering the amino acids, the ratio of the differential peptide fragments matching the amino acids meets a matching ratio threshold.

Further, the matching ratio threshold is greater than or equal to 75%.

Further, the epitope screening condition in the third region screening module 50 includes at least one of the following: (a) overlapping with the second conserved motif region; (b) a comparison score with a human proteome sequence being lower than a comparison threshold; and (c) meeting a plurality of the following performance indexes: 1) the covering number of the differential peptide fragment being ≥3; 2) hydrophilicity meeting a hydrophilic threshold; and 3) an accessibility score, a Beta turn and a multi-alignment score being all in the top 100. That the comparison score is lower than the comparison threshold means that a/b ≤ 0.8, where a is a matching score that a sequence of a region to be screened is aligned with the human proteome sequence, and b is a matching score that the sequence of the region to be screened is aligned with all proteome sequences of the target coronavirus.

Further, the third region screening module includes: a merging module, configured to merge the predicted epitope region and the first conserved motif region according to one of the following merging conditions: 1) there is an inclusion relation between the two regions; and 2) the two regions are predicted as antigen epitope regions by at least two different methods, to obtain a first candidate epitope region; an overlap screening module, configured to screen a region overlapping with the second conserved motif region from the first candidate epitope region as a second candidate epitope region; a comparison screening module, configured to screen, from the second candidate epitope region, a region of which comparison score with the human proteome sequence is lower than a first threshold, as a third candidate epitope region; a non-phosphorylation and extracellular region screening module, configured to screen and retain the non-phosphorylation region and/or the extracellular region in the proteome sequence of the target coronavirus from the third candidate epitope region, as a fourth candidate epitope region; and a comprehensive sorting module, configured to comprehensively sort the fourth candidate epitope region according to accessibility, the beta turn, the hydrophilicity, the covering number of the differential peptide fragments and a multi-alignment result, and then perform optimal selection, to obtain the antigen epitope of the target coronavirus.

Further, the device further includes: a mutation removing module, configured to remove a region including mutations from regions optimally selected by the comprehensive sorting module, to obtain the antigen epitope polypeptide of the target coronavirus.

A third aspect of the present invention provides a storage medium. The storage medium includes a stored program. When the program is operated, a device where the storage medium is located is controlled to execute the method for screening a coronavirus antigen epitope described in any one of the above.

A fourth aspect of the present invention provides a processor. The processor is configured to operate a program. When the program is operated, the method for screening a coronavirus antigen epitope described in any one of the above is executed.

Through the application of the technical solution of the present invention, by innovatively combining the polypeptide chip technology, a batch of polypeptide specifically related to coronavirus infection (especially SARS-Cov-2 virus infection). The polypeptide can be used to prepare related detection reagents such as antigens, antibodies and kits, as well as related vaccine products such as polypeptide vaccines, nucleic acid vaccines and protein recombinant vaccines. Therefore, a more powerful tool can be provided for the prevention and control of the infection and prevalence of such viruses.

### Brief Description of the Drawings

The drawings, which form a part of this application, are used to provide a further understanding of the present invention. The exemplary embodiments of the present invention and the description thereof are used to explain the present invention, but do not constitute improper limitations to the present invention. In the drawings:
Fig. 1 is a schematic flowchart of a method for screening a coronavirus antigen epitope according to a preferred embodiment of this application.
Fig. 2A and Fig. 2B respectively show the activity of serum obtained from mice immunized with different single-peptides against a neutralizing antibody produced by live coronavirus. Fig. 2A shows a detection result under a microscope, and Fig. 2B shows a statistical result.
Fig. 3A, Fig. 3B and Fig. 3C respectively show changes in an antibody signal corresponding to each polypeptide in mice immunized with a combination 1, a combination 2 and a combination 3 with time.
Fig. 4A and Fig. 4B respectively show the activity of serum obtained from mice immunized by a combination 1, a combination 2 and a combination 3 against a neutralizing antibody produced by live coronavirus. Fig. 4A shows a detection result under a microscope, and Fig. 4B shows a statistical result.
Fig. 5A to Fig. 5J respectively show changes, with time, in antibody signals corresponding to 4 polypeptides of each mix after mice are immunized with Mix1 to Mix10.
Fig. 6A to Fig. 6F show antibody production at different time points after 7 peptides are co-immunized with each adjuvant in mice.
Fig. 7 is a block diagram of a hardware structure of a method for screening an antigen epitope polypeptide according to an embodiment of the present invention.
Fig. 8 is a schematic structural diagram of a device for screening an antigen epitope polypeptide according to a preferred embodiment of this application.

### Detailed Description of the Embodiments

It is to be noted that the embodiments in this application and the features in the embodiments may be combined with one another without conflict. The present invention will be described below in detail with reference to the embodiments.

Term explanation:
"Corona Virus Disease 2019" or COVID-19 in this application refers to a disease that occurs in a patient after being infected with a SARS-Cov-2 virus (also called the novel coronavirus in this application), that is, the novel coronavirus pneumonia.

Antigen epitope is also called an antigenic determinant, which is a special chemical group with a certain composition and structure on a surface or other parts of an antigenic substance molecule, and a structure that can specifically bind to corresponding antibodies or sensitized lymphocytes. During immune response, the epitopes identified by an antigen receptor TCR of a T cell and an antigen receptor BCR of a B cell have different characteristics, which are respectively called a T cell epitope and a B cell epitope. The T cell epitope is generally not located on a surface of an antigen molecule, and can only be identified by TCR when the antibody is processed by an antigen-presenting cell into small molecular polypeptides and combined with an MHC molecule. The T cell can only identify the processed epitope. The B cell epitope may exist on the surface of the antigen molecule, and may be directly identified by the B cell without being processed. In this application, the epitope refers to one or more predicted or screened peptide fragments that can specifically bind to the antibody.

Polypeptide refers to any one predicted or screened peptide fragment that can specifically bind to the antibody or the sensitized lymphocyte.

Polypeptide-carrier protein conjugate refers to an antigen that is formed by coupling the polypeptide and a carrier protein. One carrier protein may be coupled to one or more polypeptides. When a plurality of polypeptides are coupled, the plurality of polypeptides have a same amino acid sequence. According to a difference in physical and chemical properties of a specifically coupled polypeptide sequence, different types of specific carrier proteins and different coupling methods, the number of the polypeptides coupled to each carrier protein is different, and in this application, is preferably 2-50, and more preferably, 3-45, 5-40, 5-35, 5-30, 8-30, 10-30, 12-30, or 15-30; or further preferably, the number is any one of 6-36, 8-32, 10-28, 10-26, 10-24, 10-22, 10-20, 10-18, 10-16, or 10-15.

Antigen refers to all substances that can induce an immune response in an organism, that is, the substances that can specifically not bind to the antigen receptor (TCR/BCR) on the surface of the T/B lymphocyte, activates the T/B cell to cause the T/B cell to proliferate and differentiate, so as to produce an immune response product (sensitized lymphocyte or antibody), and can specifically bind to the corresponding product in vitro and in vivo. Therefore, the antigen has two important properties: immunogenicity and immunoreactivity. The antigen in this application refers to a complete antigen with immunogenicity that is formed after polypeptide hapten is coupled to the carrier protein, which may be the polypeptide-carrier protein conjugate that is formed by coupling the polypeptide of a single amino acid sequence to the carrier protein, or may be a composition of the polypeptide-carrier protein conjugates that are formed by coupling the polypeptides with various different amino acid sequences and the carrier proteins.

A vaccine usually refers to the ability to have both immunogenicity and reactogenicity. The immunogenicity refers to performance that can stimulate the organism to produce an immune response, that is, the ability of stimulating the organism to produce a specific immune cell, causing the immune cell to activate, proliferate and differentiate, and finally produce an immunologic effector substance-specific antibody or the sensitized lymphocyte.

Polypeptide vaccine: in order to enhance the immunogenicity of the polypeptide to stimulate the organism to produce the specific antibody or the sensitized lymphocyte, a polypeptide antigen is usually immunized with an adjuvant. The commonly used adjuvants include an aluminum hydroxide adjuvant, Corynebacterium parvum, lipopolysaccharide, cytokines, or alum. A Freund's complete adjuvant and a Freund's incomplete adjuvant are the most common adjuvant in animal immunization.

A polypeptide chip technology is a detection technology based on a polypeptide chip, which uses the contact between a wide variety of polypeptides on the polypeptide chip and a sample, then uses an image acquisition technology to collect characteristic signals on the polypeptide chip (which may specifically be expressed as a fluorescent image carrying the characteristic signals), and then outputs the signal intensity of each characteristic in the chip, that is, detection result data of the polypeptide chip. By means of a sample detection signal outputted based on the detection result data of the polypeptide chip, analysis of an object to be detected in the polypeptide combined sample on the polypeptide chip and the analysis of the sample can be realized.

Motif is a data-based mathematical statistical model in biology, and may typically be a sequence or a structure, which is the sequence prediction of a specific group. For example, a DNA sequence may be defined as a transcription factor binding site. That is to say, the sequence tends to be bound by a transcription factor. For protein, a sequence motif may be defined as a protein sequence belonging to a given protein family. A simple motif may be, for example, a pattern, and the pattern is shared by all members in the group.

An ROC curve refers to a curve reflecting a relationship between sensitivity and specificity. An abscissa X-axis is 1-specificity and also called a false positive rate, the accuracy is higher when the X axis is closer to zero. An ordinate Y-axis is called sensitivity and also called a true positive rate, and if the Y-axis larger, the sensitivity is better. According to a curve position, an entire graph is divided into two parts. An area of the lower part of the curve is called an Area Under Curve (AUC), which is used to indicate prediction accuracy. If an AUC value is higher, the prediction accuracy is higher. The prediction accuracy is higher if the curve is closer to a top left corner (the smaller the X, the larger the Y).

As mentioned in the part Backgroud, an emerging coronavirus, for example, SARS-Cov-2, spreads rapidly around the world due to high infectivity. In addition, there is no target specific medicine currently, so that obtaining a corresponding vaccine as soon as possible is the key to preventing and controlling the deterioration and subsequent recurrence of the epidemic situation. Therefore, in this application, relevant research is carried out from the perspective of vaccine development, and based on research results, the technical solution of this application is proposed. This application starts with the search for novel coronavirus-specific antigen epitope. Based on an existing antigen epitope screening method and with the combination of the unique polypeptide chip technology, a batch of coronavirus family protein-related antigen epitopes is screened, and some are novel coronavirus-specific antigen epitopes. According to the polypeptide sequences corresponding to these epitopes, corresponding related products such as polypeptide antigens, detection kits, polypeptide antibodies, polypeptide vaccines and recombinant vaccines, and related products such as genetic vaccines or recombinant protein vaccines that are further developed by using these polypeptide sequences. Therefore, more ideas and means are provided for the prevention and control of coronavirus related diseases and/or COVID-19.

A preferred embodiment provides a polypeptide. The polypeptide is selected from any one of peptide fragments shown in SEQ ID NO:1 to SEQ ID NO:154 in Table 1.

A preferred embodiment provides an antigen epitope. The antigen epitope includes any one or more of SEQ ID NO:1 to SEQ ID NO:154 in Table 1.

**Table 1:**

| SEQ ID NO: | Polypeptide | Number of residues | Molecular weight | Isoelectric point | Average hydrophobicity | Name of source protein | Serial number of source protein |
|---|---|---|---|---|---|---|---|
| 1 | YTNDKACPL | 9 | 1024.1483 | 5.8279 | -0.7111 | pp1ab | YP_009724389.1 |
| 2 | RGGSYTNDKAC | 11 | 1171.2411 | 8.1973 | -1.3364 | pp1ab | YP_009724389.1 |
| 3 | SVYAWNRKR | 9 | 1179.3309 | 11.0001 | -1.4889 | Surface glycoprotein | YP_009724390.1 |
| 4 | ALDPLSETKCT | 11 | 1177.3253 | 4.3703 | -0.2545 | Surface glycoprotein | YP_009724390.1 |
| 5 | GRLQSLQTY | 9 | 1065.1803 | 8.7476 | -0.7889 | Surface glycoprotein | YP_009724390.1 |
| 6 | KVFRSSVLHSTQ | 12 | 1388.571 | 11.0008 | -0.2667 | Surface glycoprotein | YP_009724390.1 |
| 7 | GVYYPDKVFR | 10 | 1243.4094 | 8.4966 | -0.5300 | Surface glycoprotein | YP_009724390.1 |
| 8 | KRISNCVADY | 10 | 1168.3232 | 8.1973 | -0.4500 | Surface glycoprotein | YP_009724390.1 |
| 9 | NSVAYSNNS | 9 | 954.9371 | 5.5244 | -0.9111 | Surface glycoprotein | YP_009724390.1 |
| 10 | ECVLGQSKR | 9 | 1019.1766 | 8.3201 | -0.6778 | Surface glycoprotein | YP_009724390.1 |
| 11 | DYNYKLPDD | 9 | 1142.1716 | 4.1697 | -2.0333 | Surface glycoprotein | YP_009724390.1 |
| 12 | KEIDRLNEV | 9 | 1115.2375 | 4.6791 | -1.1000 | Surface glycoprotein | YP_009724390.1 |
| 13 | EVFAQVKQIY | 10 | 1224.4045 | 6.0995 | 0.1800 | Surface glycoprotein | YP_009724390.1 |
| 14 | LPFNDGVYF | 9 | 1071.1812 | 3.7999 | 0.3667 | Surface glycoprotein | YP_009724390.1 |
| 15 | NLDSKVGGNYNY | 12 | 1343.3977 | 5.8343 | -1.1750 | Surface glycoprotein | YP_009724390.1 |
| 16 | MADSNGTIT | 9 | 908.9732 | 3.7994 | -0.1556 | Membrane glycoprotein | YP_009724393.1 |
| 17 | FHPLADNKF | 9 | 1088.2152 | 6.7436 | -0.5000 | ORF7a protein | YP_009724395.1 |
| 18 | YEGNSPFH | 8 | 949.962 | 5.2402 | -1.4375 | ORF7a protein | YP_009724395.1 |
| 19 | ALNTPKDH | 8 | 894.9715 | 6.7883 | -1.3500 | Nucleocapsid phosphoprotein | YP_009724397.2 |
| 20 | KLDDKDPNFK | 10 | 1219.3436 | 6.0385 | -2.0700 | Nucleocapsid phosphoprotein | YP_009724397.2 |
| 21 | YGANKDGI | 8 | 836.8889 | 5.8349 | -0.8375 | Nucleocapsid phosphoprotein | YP_009724397.2 |
| 22 | M EVTPSGTWLTY | 12 | 1384.5525 | 3.9988 | -0.0583 | Nucleocapsid phosphoprotein | YP_009724397.2 |
| 23 | HGKEDLKF | 8 | 973.0833 | 6.7512 | -1.4750 | Nucleocapsid phosphoprotein | YP_009724397.2 |
| 24 | KKPASRELKVTF | 12 | 1403.6686 | 10.2897 | -0.8500 | pp1ab | YP_009724389.1 |
| 25 | YYKKDNSYF | 9 | 1227.3206 | 8.3788 | -1.8556 | pp1ab | YP_009724389.1 |
| 26 | NVAKSEFDRDAA | 12 | 1322.3805 | 4.5582 | -0.9000 | pp1ab | YP_009724389.1 |
| 27 | VNKGEDIQLLKS | 12 | 1343.5255 | 6.0395 | -0.5583 | pp1ab | YP_009724389.1 |
| 28 | ERSEKSYEL | 9 | 1140.2007 | 4.7864 | -2.0000 | pp1ab | YP_009724389.1 |
| 29 | LQDLKWARFPKS | 12 | 1488.7315 | 9.9943 | -0.8667 | pp1ab | YP_009724389.1 |
| 30 | ETSNSFDVLKSE | 12 | 1355.4038 | 4.4267 | -0.8500 | pp1ab | YP_009724389.1 |
| 31 | DNQDLNGNWY | 10 | 1238.2191 | 3.5637 | -1.9800 | pp1ab | YP_009724389.1 |
| 32 | KLDNYYKKDNSY | 12 | 1550.6667 | 8.3362 | -2.2167 | pp1ab | YP_009724389.1 |
| 33 | DSFKEELDKY | 10 | 1273.3446 | 4.3167 | -1.7300 | Surface glycoprotein | YP_009724390.1 |
| 34 | VYDPLQPEL | 9 | 1073.1957 | 3.6660 | -0.3556 | Surface glycoprotein | YP_009724390.1 |
| 35 | RLFRKSNLK | 9 | 1161.4002 | 12.0165 | -1.1889 | Surface glycoprotein | YP_009724390.1 |
| 36 | SNLKPFER | 8 | 990.1138 | 8.4636 | -1.4000 | Surface glycoprotein | YP_009724390.1 |
| 37 | PLQPELDSFKEE | 12 | 1431.5429 | 4.2446 | -1.2500 | Surface glycoprotein | YP_009724390.1 |
| 38 | QELGKYEQY | 9 | 1157.2293 | 4.5314 | -1.9000 | Surface glycoprotein | YP_009724390.1 |
| 39 | GTITVEELKK | 10 | 1117.2932 | 6.1425 | -0.4100 | Membrane glycoprotein | YP_009724393.1 |
| 40 | IRGGDGKMKD | 10 | 1076.2279 | 8.5901 | -1.4100 | Nucleocapsid phosphoprotein | YP_009724397.2 |
| 41 | SLPGVFCGV | 9 | 878.0467 | 5.2381 | 1.5889 | pp1ab | YP_009724389.1 |
| 42 | FLAHIQWMV | 9 | 1144.3876 | 6.7411 | 1.2667 | pp1ab | YP_009724389.1 |
| 43 | QLFFSYFAV | 9 | 1121.2829 | 5.5244 | 1.4000 | pp1ab | YP_009724389.1 |
| 44 | KLRSDVLLPL | 10 | 1153.4146 | 8.7477 | 0.5100 | pp1ab | YP_009724389.1 |
| 45 | LVAEWFLAYI | 10 | 1224.4458 | 3.9997 | 1.7000 | pp1ab | YP_009724389.1 |
| 46 | VMVELVAEL | 9 | 1002.2254 | 3.7950 | 1.8778 | pp1ab | YP_009724389.1 |
| 47 | ILSPLYAFA | 9 | 994.1834 | 5.5244 | 1.6444 | pp1ab | YP_009724389.1 |
| 48 | GLNDNLLEI | 9 | 1000.1036 | 3.6660 | 0.1667 | pp1ab | YP_009724389.1 |
| 49 | YLFDESGEFKL | 11 | 1347.4676 | 4.1374 | -0.3364 | pp1ab | YP_009724389.1 |
| 50 | KLVNKFLAL | 9 | 1045.318 | 10.0027 | 0.9889 | pp1ab | YP_009724389.1 |
| 51 | FLKKDAPYI | 9 | 1094.3026 | 8.4975 | -0.1444 | pp1ab | YP_009724389.1 |
| 52 | FVSNGTHWFV | 10 | 1193.3092 | 6.7411 | 0.4500 | Surface glycoprotein | YP_009724390.1 |
| 53 | VDEPEEHV | 8 | 952.9612 | 3.9976 | -1.3000 | ORF3a protein | YP_009724391.1 |
| 54 | KWESGVKD | 8 | 948.0308 | 6.1922 | -1.5875 | ORF3a protein | YP_009724391.1 |
| 55 | TDTGVEHV | 8 | 856.8771 | 4.3513 | -0.4500 | ORF3a protein | YP_009724391.1 |
| 56 | LLYDANYFL | 9 | 1131.2762 | 3.7999 | 0.7111 | ORF3a protein | YP_009724391.1 |
| 57 | GYTEKWES | 8 | 999.0312 | 4.5314 | -1.8750 | ORF3a protein | YP_009724391.1 |
| 58 | TDHSSSSD | 8 | 834.7425 | 4.5102 | -1.7625 | Membrane glycoprotein | YP_009724393.1 |
| 59 | DHSSSSDNI | 9 | 960.8988 | 4.1967 | -1.3778 | Membrane glycoprotein | YP_009724393.1 |
| 60 | NTDHSSSS | 8 | 833.7577 | 5.0767 | -1.7625 | Membrane glycoprotein | YP_009724393.1 |
| 61 | LNTDHSSS | 8 | 859.838 | 5.0767 | -1.1875 | Membrane glycoprotein | YP_009724393.1 |
| 62 | CPDGVKHV | 8 | 853.9857 | 6.7344 | -0.2125 | ORF7a protein | YP_009724395.1 |
| 63 | CQEPKLGS | 8 | 860.9751 | 5.9943 | -0.9250 | ORF8 protein | YP_009724396.1 |
| 64 | RNPANNAA | 8 | 826.8577 | 9.7501 | -1.4000 | Nucleocapsid phosphoprotein | YP_009724397.2 |
| 65 | EERLKLFDRYF | 11 | 1515.7104 | 6.2791 | -1.0455 | pp1ab | YP_009724389.1 |
| 66 | PGTAVLRQWLP | 11 | 1237.4498 | 10.1800 | 0.0364 | pp1ab | YP_009724389.1 |
| 67 | CPAVAKHDFFK | 11 | 1262.4791 | 8.2065 | -0.0182 | pp1ab | YP_009724389.1 |
| 68 | LQDLKWARFPK | 11 | 1401.6542 | 9.9943 | -0.8727 | pp1ab | YP_009724389.1 |
| 69 | LLTKSSEYKG P | 11 | 1222.3873 | 8.4976 | -0.8455 | pp1ab | YP_009724389.1 |
| 70 | VLTLDNQDLNG | 11 | 1201.2835 | 3.5637 | -0.2727 | pp1ab | YP_009724389.1 |
| 71 | YMRSLKVPATV | 11 | 1264.5364 | 9.9943 | 0.2818 | pp1ab | YP_009724389.1 |
| 72 | SVEEVLSEARQ | 11 | 1246.3243 | 4.2519 | -0.5545 | pp1ab | YP_009724389.1 |
| 73 | KVDGVDVELFE | 11 | 1249.3661 | 4.1564 | 0.0818 | pp1ab | YP_009724389.1 |
| 74 | LTVFFDGRVDG | 11 | 1225.3495 | 4.2078 | 0.4364 | pp1ab | YP_009724389.1 |
| 75 | EYADVFHLYL | 10 | 1269.4003 | 4.3533 | 0.3600 | pp1ab | YP_009724389.1 |
| 76 | HECFVKRVDWT | 11 | 1419.6065 | 6.7429 | -0.5909 | pp1ab | YP_009724389.1 |
| 77 | STSHKLVLSVN | 11 | 1184.3425 | 8.4894 | 0.2091 | pp1ab | YP_009724389.1 |
| 78 | KDYLASGGQPI | 11 | 1148.2656 | 5.8349 | -0.4818 | pp1ab | YP_009724389.1 |
| 79 | AVLQSGFRK | 9 | 1005.1714 | 11.0010 | -0.0556 | pp1ab | YP_009724389.1 |
| 80 | MASLVLARKHT | 11 | 1226.4918 | 11.0003 | 0.3818 | pp1ab | YP_009724389.1 |
| 81 | MQNCVLKLKVD | 11 | 1290.5952 | 7.9545 | 0.1909 | pp1ab | YP_009724389.1 |
| 82 | IERYKLEGYAF | 11 | 1388.5659 | 6.1418 | -0.5000 | pp1ab | YP_009724389.1 |
| 83 | TILGSALLEDE | 11 | 1160.2715 | 3.9129 | 0.4818 | pp1ab | YP_009724389.1 |
| 84 | KLDNYYKKDNS | 11 | 1387.4935 | 8.3788 | -2.3000 | pp1ab | YP_009724389.1 |
| 85 | QLSLPVLQVRD | 11 | 1267.4741 | 6.0877 | 0.2182 | pp1ab | YP_009724389.1 |
| 86 | AWYTERSEKSY | 11 | 1419.494 | 6.1859 | -1.7636 | pp1ab | YP_009724389.1 |
| 87 | YEKLKPVLDWL | 11 | 1403.6634 | 6.0683 | -0.2727 | pp1ab | YP_009724389.1 |
| 88 | QADVEWKFYDA | 11 | 1371.4493 | 4.0280 | -0.8636 | pp1ab | YP_009724389.1 |
| 89 | NEYRLYLDAY | 10 | 1319.4177 | 4.3703 | -0.9500 | pp1ab | YP_009724389.1 |
| 90 | INVIVFDGKSK | 11 | 1219.4295 | 8.5910 | 0.3818 | pp1ab | YP_009724389.1 |
| 91 | KKPASRELKVT | 11 | 1256.4948 | 10.2897 | -1.1818 | pp1ab | YP_009724389.1 |
| 92 | KCVPQADVEW | 10 | 1174.3261 | 4.3702 | -0.4200 | pp1ab | YP_009724389.1 |
| 93 | TDVTQLYLGG | 10 | 1066.1617 | 3.7991 | 0.1300 | pp1ab | YP_009724389.1 |
| 94 | NNDYYRSLPGV | 11 | 1297.3724 | 5.8349 | -1.1273 | pp1ab | YP_009724389.1 |
| 95 | TCTERLKLFAA | 11 | 1252.4828 | 7.8871 | 0.2909 | pp1ab | YP_009724389.1 |
| 96 | NKGEDIQLLKS | 11 | 1244.3945 | 6.0690 | -0.9909 | pp1ab | YP_009724389.1 |
| 97 | ELWAKRNIKPV | 11 | 1353.6114 | 9.9959 | -0.6818 | pp1ab | YP_009724389.1 |
| 98 | EEAKTVLKKC | 10 | 1148.3735 | 8.2707 | -0.7100 | pp1ab | YP_009724389.1 |
| 99 | SFSGYLKLTDN | 11 | 1244.3496 | 5.5526 | -0.4091 | pp1ab | YP_009724389.1 |
| 100 | NVNRFNVAITR | 11 | 1303.4697 | 12.0001 | -0.2455 | pp1ab | YP_009724389.1 |
| 101 | KYFSGAM DTT | 10 | 1120.2323 | 5.8349 | -0.4800 | pp1ab | YP_009724389.1 |
| 102 | DDYFNKKDWYD | 11 | 1508.5422 | 4.3300 | -2.3636 | pp1ab | YP_009724389.1 |
| 103 | FKESPFELEDF | 11 | 1387.4885 | 4.0020 | -0.7364 | pp1ab | YP_009724389.1 |
| 104 | FAQDGNAAIS | 10 | 993.0282 | 3.7999 | 0.1000 | pp1ab | YP_009724389.1 |
| 105 | MSYLFQHANLD | 11 | 1338.4872 | 5.3151 | -0.1545 | pp1ab | YP_009724389.1 |
| 106 | AQNSVRVLQKA | 11 | 1213.387 | 11.0010 | -0.3545 | pp1ab | YP_009724389.1 |
| 107 | VDAAKAYKDYL | 11 | 1256.4034 | 5.9289 | -0.3636 | pp1ab | YP_009724389.1 |
| 108 | KGFCDLKGKYV | 11 | 1257.5007 | 9.1129 | -0.3636 | pp1ab | YP_009724389.1 |
| 109 | EDIQLLKSAY | 10 | 1179.3194 | 4.3704 | -0.2600 | pp1ab | YP_009724389.1 |
| 110 | DPAQLPAPRTL | 11 | 1178.3381 | 5.8364 | -0.5273 | pp1ab | YP_009724389.1 |
| 111 | NKHAFHTPAF | 10 | 1169.2913 | 8.7642 | -0.6900 | pp1ab | YP_009724389.1 |
| 112 | NRYLALYNKYK | 11 | 1445.6635 | 9.8232 | -1.2545 | pp1ab | YP_009724389.1 |
| 113 | NVAKSEFDRDA | 11 | 1251.3026 | 4.5582 | -1.1455 | pp1ab | YP_009724389.1 |
| 114 | KLNVGDYFV | 9 | 1054.1955 | 5.8349 | 0.2667 | pp1ab | YP_009724389.1 |
| 115 | TH LSVDTKF | 9 | 1047.1618 | 6.4061 | -0.2222 | pp1ab | YP_009724389.1 |
| 116 | NGQVFGLYKNT | 11 | 1240.3641 | 8.5909 | -0.5818 | pp1ab | YP_009724389.1 |
| 117 | VWKSYVHVVD | 10 | 1231.3987 | 6.7227 | 0.3200 | pp1ab | YP_009724389.1 |
| 118 | HPNPKGFCDLK | 11 | 1255.4452 | 8.2065 | -1.1364 | pp1ab | YP_009724389.1 |
| 119 | YRKVLLRKNGN | 11 | 1360.6072 | 11.0972 | -1.2455 | pp1ab | YP_009724389.1 |
| 120 | ATVRLQAGN | 9 | 929.0324 | 9.7950 | -0.1111 | pp1ab | YP_009724389.1 |
| 121 | ETSNSFDVLKS | 11 | 1226.2898 | 4.3704 | -0.6091 | pp1ab | YP_009724389.1 |
| 122 | LLTKGTLEPEY | 11 | 1263.4359 | 4.5314 | -0.3818 | pp1ab | YP_009724389.1 |
| 123 | TVREVLSDR | 9 | 1074.1889 | 5.7352 | -0.5889 | pp1ab | YP_009724389.1 |
| 124 | QSRNLQEFKPR | 11 | 1402.5579 | 10.8350 | -2.0636 | pp1ab | YP_009724389.1 |
| 125 | DVVECLKLSHQ | 11 | 1270.4549 | 5.3203 | 0.0091 | pp1ab | YP_009724389.1 |
| 126 | RVEKKKLDGFM | 11 | 1350.629 | 9.6998 | -0.9909 | pp1ab | YP_009724389.1 |
| 127 | KLFDRYFKYW | 10 | 1465.6945 | 9.5263 | -0.9900 | pp1ab | YP_009724389.1 |
| 128 | DAQSFLNRVCG | 11 | 1209.332 | 5.8294 | -0.1000 | pp1ab | YP_009724389.1 |
| 129 | TCFANKHADFD | 11 | 1268.3545 | 5.3603 | -0.6000 | pp1ab | YP_009724389.1 |
| 130 | HPNQEYADVF | 10 | 1219.2589 | 4.3531 | -1.1300 | pp1ab | YP_009724389.1 |
| 131 | YKQARSEDKRA | 11 | 1351.4682 | 9.6966 | -2.3455 | pp1ab | YP_009724389.1 |
| 132 | TANVNALLSTD | 11 | 1118.195 | 4.2972 | 0.2455 | pp1ab | YP_009724389.1 |
| 133 | SCKRVLNVVCK | 11 | 1248.5619 | 9.4997 | 0.4364 | pp1ab | YP_009724389.1 |
| 134 | RHINAQVAKSH | 11 | 1260.4054 | 11.0009 | -0.9364 | pp1ab | YP_009724389.1 |
| 135 | KSAGFPFNKW | 10 | 1181.3417 | 10.0027 | -0.7600 | pp1ab | YP_009724389.1 |
| 136 | IMSDRDLYDKL | 11 | 1368.5548 | 4.4290 | -0.6364 | pp1ab | YP_009724389.1 |
| 137 | KLRSDVLLPL | 10 | 1153.4146 | 8.7477 | 0.5100 | pp1ab | YP_009724389.1 |
| 138 | CLYRNRDVDTD | 11 | 1369.4601 | 4.6762 | -1.3182 | pp1ab | YP_009724389.1 |
| 139 | VGQQDGSEDNQ | 11 | 1176.1052 | 3.4924 | -1.9909 | pp1ab | YP_009724389.1 |
| 140 | IVNNWLKQLIK | 11 | 1368.6656 | 10.0027 | 0.1455 | pp1ab | YP_009724389.1 |
| 141 | ALLTKSSEYK | 10 | 1139.2987 | 8.5410 | -0.5500 | pp1ab | YP_009724389.1 |
| 142 | PLQPELDSFKE | 11 | 1302.4289 | 4.4269 | -1.0455 | Surface glycoprotein | YP_009724390.1 |
| 143 | TSNQVAVLYQ | 10 | 1122.2282 | 5.1849 | 0.0700 | Surface glycoprotein | YP_009724390.1 |
| 144 | LIDLQELGKY | 10 | 1191.3731 | 4.3703 | -0.0200 | Surface glycoprotein | YP_009724390.1 |
| 145 | PFERDIS | 7 | 862.9263 | 4.3708 | -0.9429 | Surface glycoprotein | YP_009724390.1 |
| 146 | AHFPREGVFVS | 11 | 1245.3856 | 6.7944 | 0.1636 | Surface glycoprotein | YP_009724390.1 |
| 147 | TECSNLLLQYG | 11 | 1240.3825 | 3.9984 | 0.0182 | Surface glycoprotein | YP_009724390.1 |
| 148 | KIITLKKRWQL | 11 | 1426.7913 | 11.2639 | -0.4273 | ORF3a protein | YP_009724391.1 |
| 149 | TLSYYKLGASQ | 11 | 1230.3661 | 8.1651 | -0.3000 | Membrane glycoprotein | YP_009724393.1 |
| 150 | EELKKLLEQW | 10 | 1315.5138 | 4.7864 | -1.1300 | Membrane glycoprotein | YP_009724393.1 |
| 151 | CPDGVKHVYQ | 10 | 1145.2881 | 6.7336 | -0.6500 | ORF7a protein | YP_009724395.1 |
| 152 | LFIRQEEVQEL | 11 | 1403.579 | 4.2526 | -0.2636 | ORF7a protein | YP_009724395.1 |
| 153 | KM KDLSPRWY | 10 | 1323.5622 | 9.6998 | -1.4700 | Nucleocapsid phosphoprotein | YP_009724397.2 |
| 154 | DQVILLNKHID | 11 | 1307.4949 | 5.3918 | -0.0273 | Nucleocapsid phosphoprotein | YP_009724397.2 |

In a more preferred embodiment, the above antigen epitope includes any one or more of SEQ ID NO:25, SEQ ID NO:28, SEQ ID NO:31, SEQ ID NO:35 and SEQ ID NO:36, and SEQ ID NO:41 to SEQ ID NO:154. The polypeptides shown in SEQ ID NO:25, SEQ ID NO:28, SEQ ID NO:31, and SEQ ID NO:35 are obtained by screening the polypeptide chip at least twice, so that the polypeptides have higher potential application values as the antigen epitopes.

The above polypeptides act as the antigen epitopes specifically identified by the B cell or the T cell, and may be prepared into polypeptide vaccines to stimulate the organism to produce specific antibodies or sensitized lymphocytes (immunogenicity). During the immunizing of the organism, in order to better stimulate an immune response, an adjuvant is often added to stimulate the organism to produce a helper T cell, so as to further induce a B cell immune response. Definitely, the individual polypeptides may also be used to stimulate the immunized organism to produce the immune response.

The above polypeptide may also be prepared into an antigen, to stimulate the organism to produce antibodies. In order to better stimulate to achieve an adequate immune response (that is, the immunogenicity is very low), the using of a carrier protein with many antigen epitopes facilitates the stimulation of the helper T cell, to further induce the B cell immune response.

Therefore, a preferred embodiment further provides a polypeptide-carrier protein conjugate. The polypeptide-carrier protein conjugate includes any one of the above polypeptides and the carrier protein coupled to the polypeptide. The polypeptide-carrier protein conjugate generally acts as the antigen to detect the antibody, or acts as the antigen to prepare the antibody by immunizing an animal. Since the polypeptide can specifically identify the coronavirus, especially a SARS-CoV-2 virus, the polypeptide-carrier protein conjugate can specifically identify the antibody of the coronavirus, especially the antibody of the SARS-CoV-2 virus.

According to a preparation requirement of the polypeptide-carrier protein conjugate, the specific and appropriate carrier protein may be selected to form the polypeptide-carrier protein conjugate. The carrier protein in this application includes, but is not limited to, Bovine Serum Albumin (BSA), Ovalbumin (OVA), Keyhole Limpet Hemocyanin (KLH), or Casein (CS). According to an amino acid sequence composition of different polypeptides, in order to facilitate coupling with the carrier protein, the polypeptides required to be coupled to the carrier protein by using a linker sequence (which is also called a connexon or a linker). In this application, the linker sequence is preferably CGSG.

According to the physical and chemical properties of polypeptide amino acids, different the carrier proteins used and different coupling methods, the number of the polypeptides that can be coupled to each carrier protein is different. By comprehensively considering the efficiency of coupling and the ability of antibody recognition and binding, preferably, the number of the polypeptides coupled to each carrier protein is 2-50, and more preferably, 3-45, 5-40, 5-35, 5-30, 8-30, 10-30, 12-30, or 15-30; or further preferably, the number is any one of 6-36, 8-32, 10-28, 10-26, 10-24, 10-22, 10-20, 10-18, 10-16, or 10-15.

A preferred embodiment further provides an antigen. The antigen includes a polypeptide-carrier protein conjugate or a composition of a plurality of different polypeptide-carrier protein conjugates. The polypeptide-carrier protein conjugate is any one of the above polypeptide-carrier protein conjugates.

It is to be noted that, in the above polypeptide-carrier protein conjugate, the polypeptides coupled to the carrier protein are polypeptides having a same amino acid sequence. That is to say, the same carrier protein is coupled to the same polypeptides, so that the polypeptide-carrier protein conjugate has a single antigen epitope when acting as the antigen. In certain embodiments, when acting as the antigen to detect whether there is serum in a virus antibody, the antigen may be an antigen having the single antigen epitope, or may be an antigen having a plurality of antigen epitopes. When the polypeptide-carrier protein conjugate coupled to different polypeptide sequences acts as the antigen in the form of a composition, the plurality of antigen epitopes may be produced. For example, if an A-BSA conjugate is obtained by coupling the polypeptide of a sequence A to the BSA, a B-BSA conjugate is obtained by coupling the polypeptide of a sequence B to the BSA, and a C-OVA conjugate is obtained by coupling the polypeptide of a sequence C to the OVA, the antigen including the three polypeptide-carrier protein conjugates has A, B and C antigen epitopes. If the antigen only includes one of the three polypeptide-carrier protein conjugates, the antigen only has one antigen epitope.

A preferred embodiment further provides a detection kit for a coronavirus antibody. The kit includes any one of the above antigens. The antigen epitope of the antigens are from any one of the above polypeptides. Known coronavirus protein families all have the above polypeptides. Therefore, the kit including the antigen can accurately and specifically identify and diagnose the coronavirus, especially a patient infected with SARS-CoV-2.

The kit may be prepared into detection kits of a plurality of different types according to specific requirements. However, for easy of detection and determination of detection results, most of the polypeptide antigens in the kit are pre-coated antigens. Preferably, the pre-coated antigen is coated on a solid phase carrier, and the specific pre-coated solid phase carrier is rationally designed according to requirements. More preferably, the solid phase carrier includes an ELISA plate (which is mostly a polystyrene material), a membrane carrier or microsphere. Further preferably, the membrane carrier includes a nitrocellulose membrane (which is most widely used), a glass cellulose membrane or a nylon membrane. Further preferably, the membrane carrier is also coated with a positive control. The polypeptide-carrier protein conjugate and the positive control are successively arranged on the nitrocellulose membrane according to a detection order.

According to different specific detection methods of the kit, specific supporting reagents in the kit are different accordingly, but may be combined according to preparation methods of known kits. Preferably, the above kit also includes one of the following: (1) an enzyme-labeled secondary antibody, more preferably, the enzyme-labeled secondary antibody being an HRP-labeled secondary antibody (corresponding to an ELISA detection kit); (2) a colloidal gold bonding pad, coated with a colloidal gold-labeled specific conjugate (corresponding to an immune colloidal gold detection kit) of the polypeptide-carrier protein conjugate and the positive control; and (3) a labeling pad, coated with fluorescently labeled microsphere, the microsphere being loaded with the specific conjugate (corresponding to an immunofluorescence detection kit) of the positive control.

The immune colloidal gold detection kit and the immunofluorescence detection kit are relatively convenient in detection, which only need to establish a C line of the positive control and a T line of a detection sample. As long as the pre-coated positive control at the C line of the positive control can bind with the specific conjugate with a detection label carried during serum chromatography of a sample to be detected, the specific antigen or antibody of the specific positive control is not specifically limited. Preferably, the positive control is selected from murine immunoglobulin, human immunoglobulin, ovine immunoglobulin or rabbit immunoglobulin; and accordingly, the specific conjugate of the positive control is selected from anti-murine immunoglobulin, anti-human immunoglobulin, anti-ovine immunoglobulin or anti-rabbit immunoglobulin.

According to different immune objects, the anti-murine immunoglobulin may be the anti-murine immunoglobulin of goats or the anti-murine immunoglobulin of rabbits, or the anti-murine immunoglobulin of other immune animals. Likewise, according to different immune animals, the anti-human immunoglobulin, anti-ovine immunoglobulin or anti-rabbit immunoglobulin may also be immunoglobulin from different species. The immunoglobulin may be any one of IgM, IgG, IgA, IgD, or IgE. These anti-immunoglobulin antibodies may be monoclonal antibodies or polyclonal antibodies.

In the kit, according to the number of samples required to be detected, the specification of the ELISA plate used is different, which may be rationally selected from 12 to 384 well ELISA plate. In the pre-coated ELISA plate, according to different antigen epitopes in different polypeptide-carrier protein conjugates, or different detection objects at different onset stages, the coating amount of the polypeptide-carrier protein conjugate in each well is also different. In certain embodiments of this application, the coating amount of the polypeptide-carrier protein conjugate in each well is preferably 0.1-32 µg; preferably, 0.2-30 µg, 0.3-30 µg, 0.4-28 µg, 0.6-25 µg, 0.6-24 µg, 0.7-24 µg, 0.7-22 µg, or 0.7-20 µg; more preferably, 0.7-19 µg, 0.7-18 µg, 0.7-17 µg, 0.7-16 µg, 0.7-15 µg, 0.7-14 µg, 0.7-13 µg, or 0.7-12 µg; and further preferably, 0.8-19 µg, 0.8-18 µg, 0.8-17 µg, 0.8-16 µg, 0.8-15 µg, 0.8-14 µg, 0.8-13 µg, 0.8-12 µg, 0.8-11 µg, 0.8-10 µg, 0.8-9 µg, 0.8-8 µg, 0.8-7 µg, 0.8-6 µg, 0.8-5 µg, 0.8-4 µg, 0.8-3 µg, 0.8-2 µg, 0.8-1.8 µg, 0.8-1.7µg, 0.8-1.6 µg, 0.8-1.5 µg, 0.8-1.4 µg, or 0.8-1.2 µg.

Similarly, the coating amount of the polypeptide-carrier protein conjugate on the membrane carrier (for example, the nitrocellulose membrane) is also different, preferably 0.8-8 µg/cm, and more preferably 0.8-7 µg/cm, 0.8-6 µg/cm, 0.8-5 µg/cm, 0.8-4 µg/cm, 0.8-3 µg/cm, 0.8-2 µg/cm, 0.8-1.8 µg/cm, 0.8-1.7 g µg/cm, 0.8-1.6 µg/cm, 0.8-1.5 µg/cm, 0.8-1.4 µg/cm, or 0.8-1.2 µg/cm.

A preferred embodiment further provides applications of the polypeptide or the antigen epitope in preparation of drugs for treating related diseases caused by a coronavirus. In some preferred embodiments, the coronavirus is SARS-CoV-2. For example, the polypeptide-carrier protein conjugate including these polypeptides or the antigen epitopes is used as the antigen to immunize an animal, so as to prepare a specific antibody. Or according to the related antigen epitope provided in this application, a related polypeptide vaccine may be prepared by means of chemical synthesis. Or a nucleic acid encoding the polypeptide is obtained by using a recombinant gene, so as to obtain a genetic vaccine. Therefore, the above drug may be an antibody or a vaccine. The antibody may be the monoclonal antibody or the polyclonal antibody. The vaccine may be the polypeptide vaccine or the genetic vaccine.

Correspondingly, a preferred embodiment further provides the above drug. The drug may be an antibody or a vaccine. The antibody is obtained by immunizing an animal with the above antigen. The vaccine is a polypeptide vaccine or a genetic vaccine. The polypeptide vaccine includes any one or more of the polypeptides in Table 1. The genetic vaccine includes nucleic acids encoding any one or more of the polypeptides in Table 1. Preferably, the polypeptides are selected from any one or more of SEQ ID NO:1 to SEQ ID NO:40; and more preferably, the polypeptides are selected from any one or more of SEQ ID NO:25, SEQ ID NO:28, SEQ ID NO:31, SEQ ID NO:35 and SEQ ID NO:36. The 5 polypeptides are obtained by independently screening a polypeptide chip at least twice, so that the polypeptides are more likely to be used as vaccines in terms of probability.

It is to be noted that, the antibody is obtained by using the polypeptide-carrier protein conjugate as the antigen to immunize the animal. Commonly used immune animals include mammals such as rats, mice, goats or rabbits. According to different types of the polypeptide-carrier protein conjugates included in the antigen, the obtained antibody may be a monoclonal antibody or a polyclonal antibody. The vaccine may be a polypeptide vaccine. The polypeptide vaccine may be obtained by means of chemical synthesis according to a polypeptide sequence, or may be obtained through enzymatic digestion and purification after in vitro recombinant expression by means of genetic engineering. The genetic vaccine is designed by means of genetic engineering to include a nucleic acid encoding a target polypeptide, to cause the nucleic acid to express so as to produce the polypeptide with an antigen epitope effect.

A preferred embodiment further provides a method for preventing or treating pneumonia caused by a coronavirus. The prevention method includes giving a subject a prophylactically effective amount of an anti-coronavirus drug. The drug is the vaccine in the above drug. The treatment method includes giving the subject therapeutically effective amount of the anti-coronavirus drug. The drug is the antibody in the above drug.

Preferably, the coronavirus is SARS-CoV-2.

In this application, in order to further enhance an immune response produced due to the stimulation of the polypeptide to an organism, a preferred embodiment provides a polypeptide composition. The polypeptide composition includes at least two of peptide fragments shown in SEQ ID NO:1 to SEQ ID NO:154 in Table 1.

In certain preferred embodiments, the polypeptide composition includes at least any one of the peptide fragments shown in SEQ ID NO:1 to SEQ ID NO:40. Preferably, the polypeptide composition includes at least any one of SEQ ID NO:25, SEQ ID NO:28, SEQ ID NO:31, SEQ ID NO:35, or SEQ ID NO:36.

According to different research and development requirements such as vaccine or antibody preparation, the polypeptide compositions may be mixed in physical form to form a composition, or may be connected by using chemical bonds to form a composition in the form of long chain polypeptides. A specific connected peptide fragment sequence, number and sequential order may be rationally adjusted according to actual requirements. Preferably, connection is achieved by using two peptide fragments. A specific way of connection may be implemented by using a linker arm (which may be, for example, glycine or lysine).

In some preferred embodiments, the polypeptide composition includes one or more peptide fragments in a first peptide fragment set. The first peptide fragment set includes the peptide fragments shown in SEQ ID NO:1-4, 6-8, 11, 13-17, 20-25, 27-30, 32-33, 35-36, and 39-40. The peptide fragments in the first peptide fragment set show stronger sequence specificity to the novel coronavirus. The preparation of a vaccine on the basis of these polypeptides facilitates the obtaining of a vaccine specifically targeting the novel coronavirus.

In some other preferred embodiments, the polypeptide composition includes one or more peptide fragments in a second peptide fragment set. The second peptide fragment set includes the peptide fragments shown in SEQ ID NO:5, 9, 10, 12, 18, 19, 26, 31, 34, 37, and 38. The peptide fragments in the second peptide fragment set show stronger sequence conservation to the coronavirus. The preparation of a vaccine on the basis of these polypeptides facilitates the obtaining of a broad-spectrum vaccine for the coronavirus.

In certain embodiments, the polypeptide composition also includes, in addition to one or more peptide fragments in the first peptide fragment set, one or more peptide fragments in the second peptide fragment set. A vaccine is prepared on the basis of the peptide fragments in the above two sets, to obtain a vaccine with stronger immunogenicity against various coronaviruses.

In some embodiments, the polypeptide composition may also be formed by combining a T cell epitope and a B cell epitope, so that an immune effect can be enhanced. Specifically, whether the above 40 polypeptides are from the T cell epitope or the B cell epitope may be distinguished according to multiple epitope prediction software.

In some embodiments, the polypeptide composition includes the polypeptides derived from a same protein and/or different proteins. More preferably, there are no more than two polypeptides derived from the same protein in the polypeptide composition. Further preferably, the polypeptide composition is selected from one of the following combinations:
A combination 1: SEQ ID NO:28, SEQ ID NO:6, SEQ ID NO:13, and SEQ ID NO:18.
A combination 2: SEQ ID NO:27, SEQ ID NO:14, SEQ ID NO:5 and SEQ ID NO:17.
A combination 3: SEQ ID NO:32, SEQ ID NO:4, SEQ ID NO:10 and SEQ ID NO:23.
A combination 4: SEQ ID NO:25, SEQ ID NO:3, SEQ ID NO:34 and SEQ ID NO:40.
A combination 5: SEQ ID NO:30, SEQ ID NO:8, SEQ ID NO:37 and SEQ ID NO:21.
A combination 6: SEQ ID NO:2, SEQ ID NO:11, SEQ ID NO:33 and SEQ ID NO:19.
A combination 7: SEQ ID NO:1, SEQ ID NO:15, SEQ ID NO:12 and SEQ ID NO:29.
A combination 8: SEQ ID NO:26, SEQ ID NO:35, SEQ ID NO:38 and SEQ ID NO:22.
A combination 9: SEQ ID NO:31, SEQ ID NO:36, SEQ ID NO:16 and SEQ ID NO:20.
A combination 10: SEQ ID NO:7, SEQ ID NO:9, SEQ ID NO:39 and SEQ ID NO:24.
A combination 11: SEQ ID NO:29, SEQ ID NO:35, SEQ ID NO:40 and SEQ ID NO:20.
A combination 12: SEQ ID NO:3, SEQ ID NO:20, SEQ ID NO:21, SEQ ID NO:22, SEQ ID NO:29, SEQ ID NO:33, and SEQ ID NO:34.

In order to further effectively control the infection of the coronavirus to humans, a preferred embodiment of this application provides a polypeptide vaccine. The polypeptide vaccine includes any one or more of peptide fragments shown in SEQ ID NO:1 to SEQ ID NO:154 in Table 1. By using these polypeptides, specific peptide fragments may be rationally selected to form the effective polypeptide vaccine according to the broad-spectrum and/or novel coronavirus-specific peptide fragments.

In some preferred embodiments, the polypeptide vaccine includes at least any one of the peptide fragments shown in SEQ ID NO:1 to SEQ ID NO:40. Preferably, the polypeptide vaccine includes at least one of SEQ ID NO:25, SEQ ID NO:28, SEQ ID NO:31, SEQ ID NO:35, or SEQ ID NO:36.

In some preferred embodiments, the polypeptide vaccine includes one or more peptide fragments in the first peptide fragment set. The first peptide fragment set includes the peptide fragments shown in SEQ ID NO: 1-4, 6-8, 11, 13-17, 20-25, 27-30, 32-33, 35-36, and 39-40. The peptide fragments in the first peptide fragment set show stronger sequence specificity to the novel coronavirus.

In some other preferred embodiments, the polypeptide vaccine includes one or more peptide fragments in the second peptide fragment set. The second peptide fragment set includes the peptide fragments shown in SEQ ID NO: 5, 9, 10, 12, 18, 19, 26, 31, 34, 37, and 38. The peptide fragments in the second peptide fragment set show stronger sequence conservation to the coronavirus.

In certain embodiments, the polypeptide vaccine also includes, in addition to one or more peptide fragments in the first peptide fragment set, one or more peptide fragments in the second peptide fragment set. A vaccine is prepared on the basis of the peptide fragments in the above two sets, to obtain a vaccine with stronger immunogenicity against various coronaviruses.

The preparation of a vaccine by using coronavirus broad-spectrum polypeptides facilitates the development of a general vaccine for the coronavirus, so that the different coronavirus infections can be prevented. The vaccine prepared by using the novel coronavirus-specific polypeptides can specifically target the novel coronavirus.

In some embodiments, the polypeptide vaccine may also be formed by combining the epitope from the T cell and the epitope from the B cell, so that the combined polypeptide vaccine facilitates the enhancement of the immune effect. Specifically, whether the above 40 polypeptides are from the T cell epitope or the B cell epitope may be distinguished according to multiple epitope prediction software.

In some embodiments, the polypeptide vaccine includes the polypeptides derived from different proteins. More preferably, there are no more than two polypeptides derived from the same protein in the polypeptide vaccine. Further preferably, the polypeptide vaccine is selected from any one of the following combinations:
A combination 1: SEQ ID NO:28, SEQ ID NO:6, SEQ ID NO:13, and SEQ ID NO:18.
A combination 2: SEQ ID NO:27, SEQ ID NO:14, SEQ ID NO:5 and SEQ ID NO:17.
A combination 3: SEQ ID NO:32, SEQ ID NO:4, SEQ ID NO:10 and SEQ ID NO:23.
A combination 4: SEQ ID NO:25, SEQ ID NO:3, SEQ ID NO:34 and SEQ ID NO:40.
A combination 5: SEQ ID NO:30, SEQ ID NO:8, SEQ ID NO:37 and SEQ ID NO:21.
A combination 6: SEQ ID NO:2, SEQ ID NO:11, SEQ ID NO:33 and SEQ ID NO:19.
A combination 7: SEQ ID NO:1, SEQ ID NO:15, SEQ ID NO:12 and SEQ ID NO:29.
A combination 8: SEQ ID NO:26, SEQ ID NO:35, SEQ ID NO:38 and SEQ ID NO:22.
A combination 9: SEQ ID NO:31, SEQ ID NO:36, SEQ ID NO:16 and SEQ ID NO:20.
A combination 10: SEQ ID NO:7, SEQ ID NO:9, SEQ ID NO:39 and SEQ ID NO:24.
A combination 11: SEQ ID NO:29, SEQ ID NO:35, SEQ ID NO:40 and SEQ ID NO:20.
A combination 12: SEQ ID NO:3, SEQ ID NO:20, SEQ ID NO:21, SEQ ID NO:22, SEQ ID NO:29, SEQ ID NO:33, and SEQ ID NO:34.

In any one of the above polypeptide combinations, the mass of each polypeptide may be rationally set according to immunogenicity. In certain preferred embodiments, the mass of each polypeptide is 0.1-1 mg, preferably, 0.25-0.5 mg. After these polypeptides are physically mixed according to a rationally-optimized mass ratio, according to the volume of the mixed polypeptide combination, the polypeptide vaccine may be formed after an adjuvant is mixed in equal volume, so as to immunize an experimental animal or a human body.

In order to further enhance the immunogenicity of the polypeptide vaccine, the polypeptides may be coupled to a carrier protein. In certain preferred embodiments, the polypeptide vaccine further includes the carrier protein. A polypeptide-carrier protein conjugate is formed by coupling the polypeptides derived from different proteins and the carrier protein. A polypeptide mixture is formed after the polypeptides of any one of the above combinations are mixed according to a rational mass ratio. The polypeptide mixture is simultaneously coupled to the same carrier protein. Therefore, the polypeptide-carrier protein conjugate coupled to a plurality of polypeptide sequences can be obtained. The specific type of the carrier protein is not limited, and includes, but is not limited to, BSA, OVA, KLH, or Casein CS. In certain embodiments, the polypeptide may further be coupled to the carrier protein by means of a linker sequence. The linker sequence is preferably CGSG.

Further preferably, the polypeptide vaccine is an injection. Preferably, the injection also includes an adjuvant. More preferably, the volume of the adjuvant in the injection equals the volume of 50-100 µg of the polypeptide-carrier protein conjugate.

It is to be noted that, for easy of storage, the polypeptide combination (mixture) or a conjugate formed by coupling the polypeptide combination and a carrier may be preserved in the form of solid powder before being mixed with the adjuvant to immunize the organism. During immunization, a liquid is prepared, and then the equal volume of adjuvant is added to form the injection for immunization. Definitely, a vaccine in a liquid form may also be prepared directly with the adjuvant.

In order to further improve the affinity of certain peptide fragments, in some preferred embodiments, any peptide fragment in the polypeptide vaccine is a modified peptide fragment. Preferably, the modified peptide fragment is to add 1-4 hydrophilic amino acids at an N terminus, a C terminus or N and C termini. Preferably, the hydrophilic amino acid is Glu, Lys, Ser, or Gly. Preferably, 1-4 hydrophilic amino acids are selected from any one of Glu-Glu, Lys-Lys, or Ser-Gly-Ser.

In certain embodiments, in order to better achieve the directional coupling of the peptide fragments, any peptide fragment in the polypeptide vaccine may be a peptide fragment modified by cysteine. Specifically, it includes, but is not limited to, adding the cysteine at the N terminus, the C terminus or the N and C termini of the peptide fragment, or adding the cysteine in the middle of a peptide chain of the peptide fragment. When the cysteine is added in the middle of the peptide chain of the peptide fragment, one or more cysteines may be inserted in the middle of the peptide chain (that is, inserted between two amino acid residues), or may be linked to the middle (that is, a side chain of an amino acid in the middle of the peptide chain) of the peptide chain in a branched-chain form.

The polypeptide in the polypeptide vaccine may be in the form of a single peptide fragment, or may be in the form of a combination of a plurality of peptide fragments. In order to further improve the immunogenicity and immunoreactivity of the polypeptide vaccine, in a preferred embodiment of this application, the polypeptide vaccine includes the plurality of peptide fragments. The plurality of peptide fragments are connected in series. Preferably, at least one peptide fragment in the polypeptide vaccine is connected in series for 1-5 times, preferably, 1-3 times. More preferably, the plurality of peptide fragments are connected in series by using a linker arm. Further preferably, the linker arm is selected from glycine, lysine, (2-aminoethoxy) acetic acid (AEA), 5-aminovaleric acid (Ava), 3-amino-3-(2-nitrophenyl)propanoic acid (ANP), 3-amino-3(2-nitrobenzene) propionic acid), β-alanine, 4-aminobutyric acid (GABA), or polyethylene glycol (PEG). By means of the polypeptides connected by the PEG, not only the solubility may be enhanced, but also the polypeptides may be protected from being cleaved by a proteolytic enzyme, thereby prolonging the half-life period of biological activity.

A preferred embodiment further provides applications of any one of the above polypeptides in preparation of vaccines for treating diseases caused by coronaviruses. Preferably, the coronavirus is SARS-CoV-2.

In some preferred embodiments, the vaccine includes any one of the peptide fragments shown in SEQ ID NO: 1 to SEQ ID NO:40. More preferably, the vaccine includes any one of SEQ ID NO:25, SEQ ID NO:28, SEQ ID NO:31, SEQ ID NO:35, or SEQ ID NO:36.

In some preferred embodiments, the vaccine includes one or more peptide fragments in the first peptide fragment set. The first peptide fragment set includes the peptide fragments shown in SEQ ID NO: 1-4, 6-8, 11, 13-17, 20-25, 27-30, 32-33, 35-36, and 39-40.

In some other preferred embodiments, the vaccine includes one or more peptide fragments in the second peptide fragment set. The second peptide fragment set includes the peptide fragments shown in SEQ ID NO: 5, 9, 10, 12, 18, 19, 26, 31, 34, 37, and 38.

In some other preferred embodiments, the vaccine includes at least one peptide fragment in the first peptide fragment set and at least one peptide fragment in the second peptide fragment set.

In a preferred embodiment, this application further provides a nucleic acid vaccine. The nucleic acid vaccine includes a nucleic acid. The nucleic acid encodes any one of the above polypeptides or the polypeptide composition. Specifically, the nucleic acid vaccine may be a DNA vaccine or an RNA vaccine, more preferably, an mRNA vaccine.

In a preferred embodiment, this application further provides a recombinant protein vaccine. The recombinant protein vaccine includes any one or more peptide fragments shown in SEQ ID NO:1 to SEQ ID NO:154. Preferably, the recombinant protein vaccine is a protein vaccine that is formed by recombining any one or more peptide fragments shown in SEQ ID NO:1 to SEQ ID NO:40, more preferably, any peptide fragment shown in SEQ ID NO:25, shown in SEQ ID NO:28, shown in SEQ ID NO:31, shown in SEQ ID NO:35 and shown in SEQ ID NO:36, with 4-6 histidines or 4 Gly and 1 Ser (that is, 4 Gly and 1 Ser are connected in order).

A preferred embodiment provides a method for preventing diseases caused by coronaviruses. The prevention method includes giving a subject a prophylactically effective amount of any one of the above polypeptide vaccine, the genetic vaccine or the recombinant protein vaccine. A method for treating diseases caused by coronaviruses is further provided. The treatment method includes giving a subject a therapeutically effective amount of any one of the above antibodies.

Preferably, the coronavirus is SARS-CoV-2.

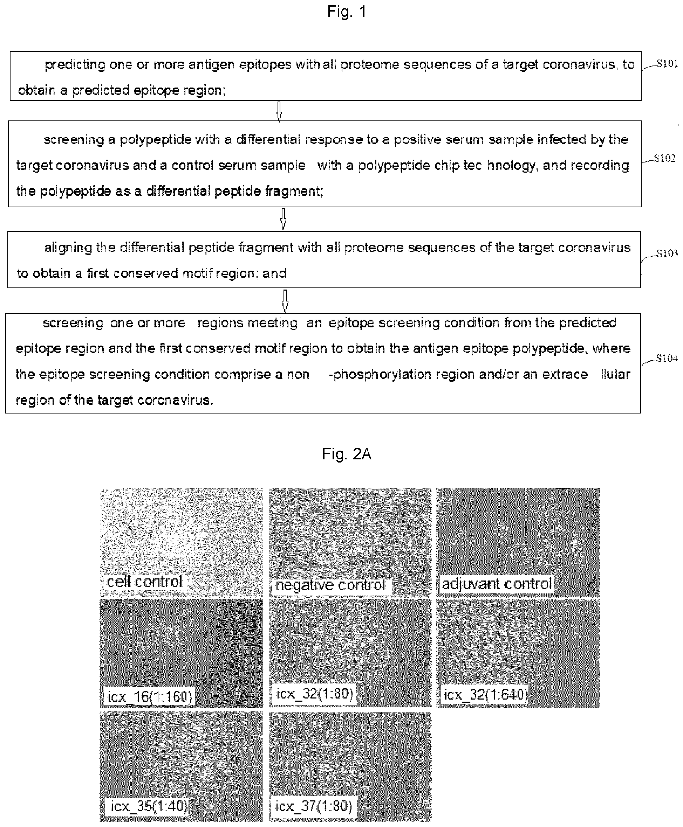

A preferred embodiment provides a method for screening an antigen epitope. As shown in Fig. 1, the screening method includes the following steps.

At S101, all proteome sequences of a target coronavirus are used to perform antigen epitope prediction, to obtain a predicted epitope region.

At S102, a polypeptide chip technology is used to screen a polypeptide with a differential response to a positive serum sample infected by the target coronavirus and a control serum sample, and the polypeptide is recorded as a differential peptide fragment.

At S103, the differential peptide fragment is aligned with all proteome sequences of the target coronavirus to obtain a first conserved motif region.

At S104, regions meeting epitope screening conditions are screened from the predicted epitope region and the first conserved motif region to obtain the antigen epitope. The epitope screening conditions include a non-phosphorylation region and/or an extracellular region of the target coronavirus.

The solution of this application and beneficial effects thereof are described below with reference to more detailed embodiments.

It is to be noted that, the antibodies, reagents and consumable materials used in the following embodiments are all commercially available products unless otherwise specified.

Unless otherwise specified, a PBS solution refers to a phosphate buffer solution (pH=7.4) with a concentration being 10 mM, and is derived from TaKaRa with an article number: T900. Polypeptide antigen diluent is a 50mM carbonate buffer solution (pH=9.6). A washing buffer solution (PBST) is prepared by adding 1 ml of tween-20 to 1 L of PBS and then performing well mixing. An HRP labeled goat anti-human IgM secondary antibody is derived from Sigma with an article number: A6907. An HRP labeled goat anti-human IgG secondary antibody is derived from Abcam with an article number: ab97225.

First portion: A method for screening an antigen epitope (that is, a polypeptide) is indicated by using a SARS-CoV-2 virus as an example.

In the prior art, a method for screening an antigen epitope is generally to, according to a target protein sequence, use public software to perform bioinformatic prediction or select according to prior knowledge. In this application, the method used has the following improvements. 1) Different software is used for comprehensive forecast evaluation, to avoid the deviation of single software; 2) a high-frequency HLA database of Chinese populations is used to assist in selection of candidate regions suitable for the Chinese populations; and 3) when a protein structure and functional information are not clear, large-scale screening is performed on an entire viral proteome sequence instead of only selecting specific protein sequences for screening to reduce computation, so that all possible candidate regions on the viral proteome can be found. In addition, the screening method of this application innovatively uses a polypeptide chip technology, which is specifically embodied in that: 1) a unique polypeptide chip technology is used (that is, a large number of polypeptides synthesized on a silicon-based chip are used to combine with an antibody in a test sample, to obtain immune characterization of the test sample without bias) to perform high-throughput screening on real data to assist in screening of differentially expressed peptide fragments between COVID-19 and a control sample; 2) the found differentially expressed peptide fragments are aligned with the regions in the proteome sequence of the novel coronavirus, and a "high-confidence conserved site" (for a specific definition, refer to step (III)) is determined according to the improvement method; and 3) a candidate region of the antigen epitope is screened on the basis of a motif of the "high-confidence conserved sites".

Specific examples of steps of the screening method are as follows.
(I) In this application, on the basis of a disclosed viral protein sequence, multiple software is invoked to predict T cell and B cell presentation sequences and regions.

A SARS-Cov-2 protein sequence (GeneBank MN908947) is acquired from NCBI, with a total length being 9703 amino acids; there are 10 Open Reading Frames (ORF) in a genome; software such as NetMHCpan4.0, IEDB_recommonded, mixMHCpred, and COBEpro are used to perform MHC-1 and MHC-2 affinity prediction on a virus whole proteome sequence; and software results are summarized, and then comprehensive evaluation is performed according to comparison results of high-frequency HLA in Chinese that are obtained by 85 experiments recorded in an Allele Frequency Net Database (AFND), to obtain 2391 high-confidence viral antigen epitope regions.

In order to improve the hydrophilicity, regions with excessively high hydrophobicity (the proportion of hydrophobic amino acids is greater than 45% and a hydrophobicity score is greater than 3) are preferably removed, to obtain 1123 antigen epitope regions. In this embodiment, a screened length is set to be 10-15 AAs. When the affinity of the peptide fragment is predicted by using the software, the polypeptide within the length range has a better affinity prediction effect. In addition, the polypeptide with the length is relatively low in synthesis cost and small in difficulty, and better purity is easy to obtain (if the length of the peptide fragment is longer, the synthesis cost is higher, the synthesis difficulty is larger, and the purity is lower).

It is to be noted that, the length of the screened peptide fragment is not limited to 10-15 AAs. In some other embodiments, the length may also be set to be 8-20, 8-18, or 8-16 AAs.
(II) Differential peptide fragments are obtained on the basis of detection data of a polypeptide chip technology platform of healthy population samples and samples of patients with coronavirus infection. By aligning the differential peptide fragments with protein sequences of influenza virus, common cold related virus and more coronaviruses, the differential peptide fragments are finally determined as sequence-related peptide fragments of a coronavirus family.

70 COVID-19 serum samples (F), 5 healthy human serum samples (H) and 5 serum samples of other lung diseases (T) are collected. The polypeptide chip technology is used to screen peptide fragments corresponding to antibodies differentially expressed in the COVID-19 serum samples compared with the healthy human serum samples and the serum samples of other lung diseases.

The idea of screening is that, at step one, by means of comparison of F and H, polypeptide characteristics may be screened, such characteristics correspond to the increase of an antibody concentration caused by diseases, but the found antibodies are not necessarily specific to COVID-19, and may also be the increase of antibodies caused by factors such as pulmonary infection; and at step two, by comparing F with T, the antibodies specific to COVID-19 compared with other lung diseases may be found; however, since the expression of the antibodies is relatively complex in a disease state and the number of T samples is limited, the comparison between COVID-19 and other lung diseases may easily find some non-specific polypeptides by mistake; and finally, an intersection of the characteristic peptide fragments found in step one and step two is taken, so that high-accuracy COVID-19-specific peptide fragments are obtained.

The flow of a basic operation for screening the differential peptide fragments includes the following.

A V13 chip (which is produced by Health Tell, with a model being P/N: 600001 V13 Slides) is used to detect the samples according to a standard procedure, to obtain the signal values of 125,509 peptide fragments of the V13 chip. The signal value of each peptide fragment is called a characteristic, of which range is 0-65535, and log10 conversion is performed on raw data. Assuming that COVID-19 causes the elevation of a specific antibody signal, by means of a single-tail T test, an elevated p value of F compared with T in each characteristic is calculated, then multiple hypothesis test correction is performed (when there are more than 2 hypothesis tests on a same dataset, multiple hypothesis test correction is required to be performed), and p_FT_BH is recorded; simultaneously, an elevated p value of F compared with H in each characteristic is calculated, then multiple hypothesis test correction is performed, and p_FH_BH is recorded; and all characteristic peptide fragments that simultaneously meet p_FT_BH < 0.05 and p_FH_BH < 0.05 are screened as target peptide fragments. 864 characteristic peptide fragments with significant response differences between healthy populations and other pneumonia populations are screened.

It is to be noted that, the above screening process may also be performed on the basis of the raw data of the signal values of the peptide fragments. A difference between the signal value of the positive serum sample and the signal value of the negative control serum sample is recorded as a first difference value, and a difference between the signal value of the positive serum sample and the signal value of the control serum sample of another lung disease is calculated and recorded as a second difference value. All combined peptide fragments meeting a condition that the first difference value and the second difference value simultaneously meet a threshold are retained, so as to obtain target differential peptide fragments.

In this case, the threshold should ensure that the positive is greater than the negative and the positive is greater than other diseases, which may be 0 or a certain proportion of smaller characteristics (for example, 110%-300%). For example, the signal value of the positive is greater than that of other diseases. For example, the positive is x, and other diseases are y, it is required that x > y. However, sometimes, a detection standard is expected to be more stringent, it may also set as x > ay, and a=1.1-3.

In the above step, the differential peptide fragments are first found. For each peptide fragment value, it is required to compare the signals of the samples in different groups in pairs. There are a total of 125,509 signal peptide fragments, so that 125,509 comparisons are required, and then multiple hypothesis test correction n=125,509 is performed. Multiple hypothesis test correction is performed on the differential p value of each peptide fragment to obtain a q value, and which two groups have differences is determined according to the q value.

The p value of each protein is calculated by means of a statistical method such as the T test. The T test is a commonly used statistical method in the detection of differential protein expression. By merging variable data between the samples, whether a certain protein is differentially expressed in two samples is evaluated. However, since the sample size is usually small, the estimation of the overall variance is not very accurate. As a result, the test power of the T test is reduced, and the number of false positives is significantly increased if the T test is used for a plurality of times.

For example, when the p value of certain protein is less than 0.05 (5%), it is considered that the protein is differentially expressed in the two samples. But there is still a 5% chance that the protein is not a differential protein. Then, original hypothesis (there is no differential expression between the two samples) is wrongly denied, resulting in false positives (the probability of error is 5%). If the test is performed once, the probability of error is 5%; if the test is performed for 10000 times, the number of error is 500, that is, there are 500 more differences. That is to say, there is actually no difference. In order to control the number of false positives, multiple test correction is required to be performed on the p value, to increase the threshold.

By using BLASTp, the differential peptide fragments are aligned with protein sequences of various pathogenic microorganisms such as coronaviruses, influenza viruses, common cold-related viruses, pneumonia-related bacteria, mycoplasma, and chlamydia published in an existing database. A result shows that 443 differential peptide fragments can be directly aligned with a novel coronavirus proteome with a high alignment score (Bit score), a threshold of the bit score is 14. More than 2 overlapping comparison results in the comparison results are called a 2CCR region (more than 2 polypeptide continuous coverage regions), and 861 differential peptide fragments are located in the 2CCR region of the novel coronavirus proteome. Therefore, it indicates that almost all of these differential signal peptide fragments are from a novel coronavirus-related immune response.

The comparison score is obtained through alignment according to the rules of BLASTp. The BLASTp has a plurality of modes suitable for different scenarios. In this embodiment, a "short sequence alignment" mode is selected. The threshold 14 is the further screening (or verification) of the 864 differential peptide fragments that have differential responses in COVID-19 patients compared with the healthy population and other pneumonia patients. That is to say, the 864 differential peptide fragments that have been obtained in the previous step are inputted. 443 differential peptide fragments can be directly compared with (high score) the protein sequence of the novel coronavirus, which indicates that the results obtained by the previous screening method are reliable. For the 443 differential peptide fragments, the high-comparison score here proves to some extent that these differential peptide fragments are indeed from the novel coronavirus.

A detailed process for producing data by the polypeptide chip technology includes the following.

### 1. Experimental design

A 96-well plate is used as a detection unit. An experimental design is prepared before an experiment starts. According to the number of detection samples, the number of blank controls set and the number of standards, the number of chips required to be used is calculated, and the serial number of the chips and the layout of the samples are determined.

**Table 2:**

| cassette SN 100905 | | | | | | | | | | | | |
|---|---|---|---|---|---|---|---|---|---|---|---|---|
| | Slide 1 001752_01 | | | Slide 2 001752_02 | | | Slide 3 001752_03 | | | Slide 4 001752_04 | | |
| | 1 | 2 | 3 | 1 | 2 | 3 | 1 | 2 | 3 | 1 | 2 | 3 |
| A | F128 | F286 | **F573** | F141 | F385 | F567 | F114 | F189 | F560 | F123 | F313 | **blk** |
| B | F61 | F421 | **std** | F108 | **F573'** | F330 | **F574** | F156 | F276 | **std** | F517 | F135 |
| C | **blk** | F569 | F41 | F45 | F111 | **F574'** | F470 | **blk** | F37 | F59 | F133 | F364 |
| D | F299 | F451 | **F575**' | **std** | F562 | F80 | F9 | F91 | **F577** | F126 | F365 | F575 |
| E | F338 | F478 | F506 | F152 | F475 | **std** | F307 | TB5 | **std** | F261 | **std** | F464 |
| F | H2 | **std** | TB1 | F47 | F220 | F460 | F2 | F88 | F284 | F1 | F25 | F95 |
| G | F3 | F74 | F458 | H10 | **blk** | TB2 | F16 | F83 | F377 | **F577'** | H24 | TB4 |
| H | F5 | F71 | F308 | F18 | F84 | F495 | **std** | H8 | TB3 | F40 | F102 | H1 |

In this embodiment, a total of 4 chips are used. Codes of the chips are 001752_01, 001752_02, 001752_03, and 001752_04, respectively. 2 standards (std) and 1 blank control (blk) are set for each chip, and the rest are detection samples. 8 holes shown in bold are two replicates for 4 samples (that is, F573 and F573', F574 and F574', F575 and F575', and F577 and F577'). Those with the same number are the same sample. The standard, the blank control and the detection sample are randomly distributed on all chips used, and details are shown in Table 2.

### 2. Experimental procedure

### 1) Sample preparation

A serum or plasma sample is diluted 25 folds twice in a 96-well deep well plate by using a PBST solution containing 1% D-mannitol, to obtain a 625-fold diluted sample plate to be detected for later use.

### 2) Hydration and assembly of chips

The chips are placed in a chip hydration tool, ultra-pure water is added to cover the chips, and hydration is performed on an orbital shaker for 20 min at 55±5 rpm/min. Then, isopropyl alcohol is used to spray surfaces of the chips, and the chips are then put into a centrifuge for centrifugation and drying. The dried chips are assembled into an assay cassette according to a position of the experimental design.

### 3) Incubation and combination of samples and chips

The diluted sample is added to the assembled chip at 90 µL/well, and then placed on a constant-temperature shaker for vibration and incubation for 1 h.

### 4) Sample cleaning

The assay cassette is placed to a plate-washing machine for cleaning.

### 5) Fluorescent secondary antibody incubation

A PBST solution containing 0.75% casein is used to prepare a 2 nM fluorescent secondary antibody solution, and then the solution is added to the assay cassette at 40 µL/well and placed on the constant-temperature shaker for vibration and incubation for 1 h.

### 6) Secondary antibody cleaning

Same as step 3).

### 7) Imaging

The chips in the assay cassette are assembled into an imaging cassette after being disassembly, cleaning and drying, and then are put into an ImageXpress micro4 imager of Molecular Device for scanning and imaging. Finally, each detection sample obtains a TIFF picture file, that is, the raw data.

### 3. Data pre-processing

1) A fluorescence intensity value of a characteristic is extracted, and 1 GPR5 data file and 1 corner images file are outputted. The GPR5 file includes all information of a sample and fluorescence intensity information of all characteristics.
2) The fluorescence intensity information of the characteristics is extracted from the GPR5 data files of all samples, and an original fluorescence intensity (foreground, FG) data matrix is generated. Then, logarithmic transformation is performed on the data of each sample, to obtain a Log-Transferred Foreground (LFG) data matrix and a Normalized and Log-Transferred Foreground (NLFG) data matrix for z-score. A sample chip information file is also produced in the step. The file includes information such as a sample array position and a serial number of chips used.

### 4. Quality control

The quality control of samples and systems is performed through a quality control method of Health Tell, and the samples and the systems are qualified.
(III) A statistic model is constructed on the basis of the differential peptide fragments and the comparison results of all peptide fragments and the novel coronavirus sequence, to obtain the high-confidence conserved site of the motif.

All of the 125,509 peptide fragments of the V13 chip are compared with the proteome sequence of the novel coronavirus by using the BLASTp. By using a single amino acid as a unit, the p values of all of the peptide fragments covering the amino acid are calculated. The p values are obtained by calculating a signal difference of the peptide fragment between two groups (COVID-19 VS control). All of the peptide fragments covering the amino acid are divided into two groups: match or mismatch with the amino acid. Distribution of the p values of the peptide fragments in the match group and the mismatch group is determined (distribution is a pattern). If the p value of the peptide fragment in the match group is significantly lower than that of the mismatch group (when the distribution of the p values in two groups is compared, "Wilcoxon signed rank test" is used, and the threshold for testing significance is P<0.05), the amino acid at this position is determined as the high-confidence conserved site.

The regions where the 443 differential peptide fragments can be directly mapped to the novel coronavirus proteome are used as motif regions; then selection is performed according to the high-confidence conserved site; and finally, 136 motif regions are totally obtained, the hydrophobicity of these regions is calculated, regions (that is, regions that the proportion of the hydrophobic amino acids is less than or equal to 45% and the hydrophobicity score is less than or equal to 3, and for a method for calculating the hydrophobicity score, refer to a document A simple method for displaying the hydropathic character of a protein. (Kyte J, Doolittle RF.)) with excessively high hydrophobicity are removed, and 114 motif regions are remained.
(IV) A confidence conserved site of the motif is obtained on the basis of the comparison results of the differential peptide fragments and the sequence of the coronavirus family.

864 differential peptide fragments are mapped to the sequence of the coronavirus family. By using the single amino acid as a unit, sites of which match rate of the differential peptide fragments covering the amino acid exceeds 75% are used as the confidence conserved sites, and 350 motif regions are obtained.

The coronavirus family includes 1600 coronaviruses in total. Some coronaviruses are listed below: Bat Hp-betacoronavirus/Zhejiang2013; Betacoronavirus England 1; Betacoronavirus Erinaceus/VMC/DEU/2012; Betacoronavirus HKU24; Bovine coronavirus; Human coronavirus HKU1; Human coronavirus OC43; Middle East respiratory syndrome coronavirus; Murine hepatitis virus; Pip polypeptide chip rellus bat coronavirus HKU5; Rabbit coronavirus HKU14; Rat coronavirus Parker; Rousettus bat coronavirus; Rousettus bat coronavirus HKU9; SARS coronavirus; and Tylonycteris bat coronavirus HKU4.
(V) Final specific peptide fragments (that is, antigen epitope) are picked out on the basis of the motif sites and a predicted presentation region.

Screening is performed for three time according to the above steps; 24 peptide fragments are screened for the first time; 97 peptide fragments (2 of the 97 peptide fragments are duplicate with 2 of the 24 peptide fragments in the first batch) are screened for the second time; 40 peptide fragments (there are 5 peptide fragments duplicate with that in the second batch) are screened for the third time; there are total 154 peptide fragments; and the serial number, sequence and basic attribute of the peptide fragment are shown in Table 1, and the first 40 peptide fragments in Table 1 are selected as follow-up vaccine peptides. Details are shown in Table 3.

**Table 3:**

| SEQ ID NO: | Single-letter polypeptide | icx_ID | Specificity |
|---|---|---|---|
| 1 | YTNDKACPL | icx_2020_vaccine_38 (Abbreviated as icx_38, the same below) | Specific |
| 2 | RGGSYTNDKAC | icx_2020_vaccine_30 | Specific |
| 3 | SVYAWNRKR | icx_2020_vaccine_33 | Specific |
| 4 | ALDPLSETKCT | icx_2020_vaccine_2 | Specific |
| 5 | GRLQSLQTY | icx_2020_vaccine_11 | Broad-spectrum |
| 6 | KVFRSSVLHSTQ | icx_2020_vaccine_19 | Specific |
| 7 | GVYYPDKVFR | icx_2020_vaccine_13 | Specific |
| 8 | KRISNCVADY | icx_2020_vaccine_18 | Specific |
| 9 | NSVAYSNNS | icx_2020_vaccine_40 | Broad-spectrum |
| 10 | ECVLGQSKR | icx_2020_vaccine_6 | Broad-spectrum |
| 11 | DYNYKLPDD | icx_2020_vaccine_5 | Specific |
| 12 | KEIDRLNEV | icx_2020_vaccine_15 | Broad-spectrum |
| 13 | EVFAQVKQIY | icx_2020_vaccine_9 | Specific |
| 14 | LPFNDGVYF | icx_2020_vaccine_22 | Specific |
| 15 | NLDSKVGGNYNY | icx_2020_vaccine_25 | Specific |
| 16 | MADSNGTIT | icx_2020_vaccine_23 | Specific |
| 17 | FHPLADNKF | icx_2020_vaccine_10 | Specific |
| 18 | YEGNSPFH | icx_2020_vaccine_36 | Broad-spectrum |
| 19 | ALNTPKDH | icx_2020_vaccine_1 | Broad-spectrum |
| 20 | KLDDKDPNFK | icx_2020_vaccine_16 | Specific |
| 21 | YGANKDGI | icx_2020_vaccine_37 | Specific |
| 22 | M EVTPSGTWLTY | icx_2020_vaccine_24 | Specific |
| 23 | HGKEDLKF | icx_2020_vaccine_29 | Specific |
| 24 | KKPASRELKVTF | icx_2020_vaccine_20 | Specific |
| 25 | YYKKDNSYF | icx_2020_vaccine_39 | Specific |
| 26 | NVAKSEFDRDAA | icx_2020_vaccine_26 | Broad-spectrum |
| 27 | VNKGEDIQLLKS | icx_2020_vaccine_34 | Specific |
| 28 | ERSEKSYEL | icx_2020_vaccine_7 | Specific |
| 29 | LQDLKWARFPKS | icx_2020_vaccine_21 | Specific |
| 30 | ETSNSFDVLKSE | icx_2020_vaccine_8 | Specific |
| 31 | DNQDLNGNWY | icx_2020_vaccine_3 | Broad-spectrum |
| 32 | KLDNYYKKDNSY | icx_2020_vaccine_17 | Specific |
| 33 | DSFKEELDKY | icx_2020_vaccine_4 | Specific |
| 34 | VYDPLQPEL | icx_2020_vaccine_35 | Broad-spectrum |
| 35 | RLFRKSNLK | icx_2020_vaccine_31 | Specific |
| 36 | SNLKPFER | icx_2020_vaccine_32 | Specific |
| 37 | PLQPELDSFKEE | icx_2020_vaccine_27 | Broad-spectrum |
| 38 | QELGKYEQY | icx_2020_vaccine_28 | Broad-spectrum |
| 39 | GTITVEELKK | icx_2020_vaccine_12 | Specific |
| 40 | IRGGDGKMKD | icx_2020_vaccine_14 | Specific |

It is to be noted that, the physical and chemical properties of 154 polypeptide sequences (including the above 40 polypeptide sequences) are shown in Table 1. The species origin of all sequences is SARS-CoV-2. In Table 1 or Table 3,

Surface glycoprotein is also called an S protein.
pp1ab: orf1ab polyprotein.
Membrane glycoprotein is also called an M protein.
Nucleocapsid phosphoprotein is also called an N protein.
Specific: referring to sequences only in SARS-cov-2.
Broad-spectrum: referring to sequences shared by coronavirus family proteins.

It is to be noted that, in Table 1, performance parameters such as molecular weight, number of residues, isoelectric point, and average hydrophobicity are all predicted through software, which specifically refer to a website: https://biopython.org/.

A specific operation is as follows.

1123 antigen epitope regions obtained in (I) are connected to and merged with 114 motif regions obtained in (III). A merging standard is that: 1) There is an inclusion relation between the two regions; or 2) the two regions are predicted as the antigen epitope regions by different software, to obtain 800 candidate epitope regions; the V13 chip peptide fragments in these regions (that is, the above merged 800 candidate epitope regions) that can cover and overlap with the 350 motif regions in (IV) as candidates for vaccine peptide fragments, and 728 candidate regions are obtained in total.

Sequences of the 728 candidate regions are compared with a human proteome sequence, and a total of 540 regions with a comparison score is lower than 0.8 are retained. A non-phosphorylation region and an extracellular portion of the novel coronavirus proteome are screened to obtain 431 regions. The accessibility, beta turn, hydrophilicity, covering number of HT peptide fragments and multi-alignment result of these regions are comprehensively sorted. Comprehensive sorting specifically includes the following.

First, regions that the covering number of the differential peptide fragments is ≥ 3 are screened. A covering condition is that a BlastP alignment score (BitScore) is greater than 14 (that is, meeting conditions that the alignment score is greater than 14 and there are at least 3 differential peptide fragments covering a certain region).

Next, the regions of which hydrophilicity is lower than a hydrophilic threshold (or the hydrophobicity is higher than a first hydrophobic threshold, and the meaning of the first hydrophobic threshold is the same as the meaning of the foregoing hydrophobic threshold) are removed.

Then, the accessibility and the beta turn are sorted from high to low, and optimal selection is performed according to the multi-alignment result.

11 regions located in pp1ab are preferably selected, of which 2 regions are specific to the novel coronavirus, and 9 regions are broad-spectrum to the coronavirus. 19 regions (12 regions are specific to the novel coronavirus, and 7 regions are broad-spectrum to the coronavirus) of the S protein, 6 regions (5 regions are specific to the novel coronavirus, and 1 region is broad-spectrum to the coronavirus) of the N protein, 2 regions (specific) of the M protein and 2 regions (one is specific, and the other one is broad-spectrum) of ORF7a are selected, so as to obtain a total of 40 peptide fragments (in total, 29 are specific and 11 are broad-spectrum, and details are shown in Table 3).

Finally, according to requirements, a step of removing regions including mutations may also be included. The step is an optional step. When the existence of the type of mutation is determined, the region of a certain mutation may also be included.

When comprehensive sorting is performed according to the accessibility, beta turn, hydrophilicity, covering number of HT peptide fragments and multi-alignment result of the 431 regions, consideration is performed based on the following: since there are 10 proteins in the SARS-CoV-2 proteome, the pplab protein is the longest, and a length the pplab protein is more than ten times that of other proteins (such as S/N protein), most of the 431 regions are also located in the pplab protein sequence. Considering that the biological significance of each protein is different, the regions of the other proteins few in number are also selected first. In addition to the including of mutations, the screening indexes of the 4 regions all meet the requirements. Therefore, these regions may also serve as candidate peptide fragments when considering the design of vaccines against the presence of variants.

The alignment score that BLASTp comparison is performed on the sequences of the 728 candidate regions and the human proteome sequence is divided by a BLASTp alignment score of the sequences of the 728 candidate regions and the novel coronavirus; and a threshold of the obtained value is 0.8. That is to say, the candidate regions greater than 0.8 are removed. The score is based on a matching degree. The BLASTp is widely used alignment software provided by NCBI, and Bitscore is the score given by the software. The similar software includes DIAMOND, Muscle, and ClustalW.

Second portion: Chemical synthesis and biological validation are performed on the screened vaccine peptide fragments

After potential vaccine peptide fragment sequence information is obtained, a candidate vaccine polypeptide is produced by means of a chemical synthesis method. A quality control requirement for the polypeptide is that HPLC-MS purity is more than 98% and endotoxin content is not higher than 1 EU/mg, so as to guarantee that the polypeptide meets the requirements of an animal in vivo experiment. Biological validation and effectiveness screening are performed on a polypeptide product passing quality control by means of the animal in vivo experiment. Young and healthy mice with complete immune system functions are administrated subcutaneously, and blood samples are regularly extracted for polypeptide chip detection. Then, the immunogenicity is assessed by analyzing a difference in signal intensity of the polypeptide sequences of the specific polypeptide chips corresponding to the designed vaccine peptide fragments. In addition, mouse endpoint sera are also used for a live virus neutralization experiment (CPE method), so as to assess a neutralizing effect of antibodies in mice after immunization.

The polypeptides synthesized based on this method may be used alone or in combination, or may be used in conjunction with proteins, and may also be used in combination with different reagents. A specific solution is described by the following embodiments.

### Synthesis of polypeptide vaccine peptides

1) Optimization design of vaccine peptides:
   a. The hydrophobic amino acids at both ends of the vaccine peptide are avoided without destroying a core site of the vaccine peptide on an antigen.
   b. The hydrophobicity of the vaccine peptide after avoidance is calculated.
2) Vaccine preparation:
   The foregoing obtained 40 peptide fragments shown in SEQ ID NO:1 to SEQ ID NO:40 are directly synthesized (unmodified) by entrusting a third-party company.

Custom 8-12AA peptide preparation: a total of 40 custom peptides are shown in the following table; each peptide is 50 mg, and is divided into 5 mg/piece of preparations for a total of 10 pieces; and purity is greater than or equal to 98%, and sterilization and lyophilization are performed under GMP cleanliness requirements.

In other embodiments, according to the nature of each specific sequence, for example, the water solubility of the peptide fragment is required to be improved, Glu-Glu, Lys-Lys or Ser-Gly-Ser may be added to the N terminus, C terminus or N and C termini of the peptide fragment simultaneously before synthesis.

In other embodiments, in order to better achieve directional coupling, the peptide fragment may be modified with cysteine, including, but not limited to, simultaneously adding the cysteine at the N terminus, C terminus or N and C termini, or adding the cysteine in the middle of a chain fragment of the peptide fragment. When the cysteine is added in the middle of the peptide chain of the peptide fragment, one or more cysteines may be inserted in the middle of the peptide chain, or may be linked to the middle of the peptide chain in a branched-chain form.

### Embodiment I Effectiveness validation of single peptide vaccines

### I. Experimental operation

### 1. Immunized mice

ICX_ID:icx_16, 24, 32, 35, and 37 (respectively corresponding to SEQ ID NO.20, SEQ ID NO.22, SEQ ID NO.36, SEQ ID NO.34, and SEQ ID NO.21) are picked out to perform effectiveness validation of single peptide vaccines. In an experiment, 5 to 6-week-old female Balb/c mice are randomly divided into 6 groups, 5 of which are single peptide experimental groups (that is, respectively corresponding to icx_16, 24, 32, 35, and 37) and 1 is a simple adjuvant group. Each group has 5 mice.

The polypeptide powder synthesized under the above conditions is dissolved in a PBS solution, and diluted to prepare a polypeptide solution with a final concentration being 2 mg/ml. For a polypeptide experimental group, 100 µg of polypeptides are respectively injected to the mice in each group at Day 0, 14 and 28 according to the grouping, and a total of 300 ug of polypeptides are injected into each mouse in 3 times. During injection, the polypeptide solution and an equal volume of adjuvant MF59 (AddaVax, Invivo Gen) are mixed, and then are subcutaneously administrated to gastrocnemius muscle of two legs of mice by using a microsyringe. In the simple adjuvant group, only the MF59 original concentration solution having a same volume as a final solution of the polypeptide experimental group is used for injection. The simple adjuvant group is administered for experiment in the same manner and frequency as the polypeptide experimental group. Day 35 after the mice are immunized is used as an experimental end point. The mice are killed. Then, blood is collected and separated to prepare supernatant.

### 2. In-vitro live virus neutralization experiment with mouse serum

10-Fold dilution at a dilution of 10⁻¹-10⁻¹⁰ is continuously performed on the live coronavirus, the diluted virus is separately inoculated to a 96-well culture plate, and a column of 8 wells are inoculated for each dilution. After cell suspension is added to each well for 5 days of co-culture, the number of holes with Cytopathic Effect (CPE) is counted under a microscope, and an infective dose TCID50 of virus half cell cultures is obtained.

Complement inactivation is performed on the mouse serum at 56°C for 30 min. 1:10, 1:20, 1:40, 1:80, 1:160, 1:320, 1:640, and 1:1280 gradient dilution is performed on the mouse serum after inactivation. A solution containing 100 TCID50 viruses and the serum of each dilution are equivalently mixed, and then incubated in a 37°C water bath for 1 h. The incubated virus serum mixed solution is added to the 96-well culture plate pre-inoculated with vero cells, and then the culture plate is incubated in an environment of 37°C and 5% CO₂. After inoculation, CPE is observed every day, and a final result is determined at Day 7.

### II. Experimental result

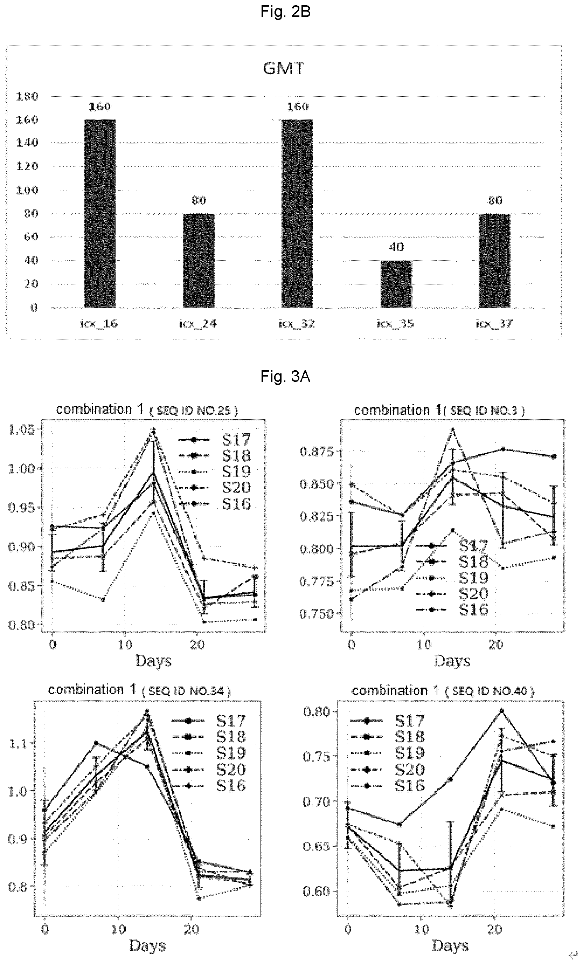

As shown in Fig. 2A, 2B and the table below, the serum obtained from the mice immunized with single peptides can have neutralizing activity to the novel coronavirus. Antibodies in mice injected with icx_32 has the optimal neutralization effect, 40% of which produces neutralizing antibodies, the highest neutralizing titer reaches 1:640, and the geometric mean neutralizing titer is 1:160. Results show that, the novel coronavirus vaccine peptides designed with the aid of the polypeptide chips can neutralize the novel coronavirus.

**Table 4:**

| Single peptide vaccine | Neutralizing antibody positive rate (%) | Highest neutralizing titer | Geometric mean neutralizing titer (GMT) |
|---|---|---|---|
| icx_16 | 20 | 1:160 | 1:160 |
| icx_24 | 20 | 1:80 | 1:80 |
| icx_32 | 40 | 1:640 | 1:160 |
| icx_35 | 20 | 1:40 | 1:40 |
| icx_37 | 40 | 1:80 | 1:80 |

### Embodiment II Effectiveness validation of combined polypeptide vaccines

### I. Experimental operation

### 1. Immunized mice

12 polypeptides are selected from the table below to form 3 polypeptide combinations. In an experiment, 5 to 6-week-old female Balb/c mice are randomly divided into 4 groups, 3 of which are polypeptide combination experimental groups (combinations 1, 2 and 3) and 1 is a simple adjuvant group. Each group has 5 mice.

40 bars of polypeptide powder are respectively dissolved and diluted to a polypeptide solution with a final concentration being 2 mg/ml by using a PBS solution. For the polypeptide combination experimental group, according to grouping information, and in each group of combinations, 50 µg of each polypeptide is mixed to form a mixed solution containing a total of 200 µg of polypeptides as a polypeptide solution for first injection in mice. Then, in each group of combinations, 25 µg of each polypeptide is mixed to form a 100 µg of the mixed solution as the polypeptide solution for second and third injections in mice. First, second and third injections are respectively and correspondingly injected to the mice in each group according to grouping at Day 0, 7 and 14. During injection, the polypeptide solution and the equal volume of adjuvant Imject Alum Adjuvant (Thermo Fisher Scientific) are mixed, and then are subcutaneously administrated to gastrocnemius muscle of two legs of mice by using a microsyringe. In the simple adjuvant group, only the Imject Alumn original concentration solution having a same volume as a final solution of the polypeptide experimental group is used for injection. The simple adjuvant group is administered for experiment in the same manner and frequency as the polypeptide experimental group.

Before the mice in each experimental group are immunized (Day 0) and at Days 7, 14, 21 after initial immunization (Days 7 and 14 are before injection), mouse tail vein blood samples are collected. The blood sample volumes collected at each time point are about 100-200 µL. Serum is prepared through separation for polypeptide chip detection. Day 28 after the mice are immunized is used as an experimental end point. The mice are killed. Then, blood is collected and separated to prepare supernatant for neutralization experiments.

**Table 5:**

| | | | | |
|---|---|---|---|---|
| Combination 1 | icx_39 (SEQ ID NO:25) | icx_33 (SEQ ID NO:3) | icx_35 (SEQ ID NO:34) | icx_14 (SEQ ID NO:40) |
| Combination 2 | icx_3 (SEQ ID NO:31) | icx_32 (SEQ ID NO:36) | icx_23 (SEQ ID NO:16) | icx_16 (SEQ ID NO:20) |
| Combination 3 | icx_21 (SEQ ID NO:29) | icx_31 (SEQ ID NO:35) | icx_14 (SEQ ID NO:40) | icx_16 (SEQ ID NO:20) |

### 2. Detection of peptide immunogenicity by polypeptide chip

### 2.1 Experimental operation

In a proactive experiment, the polypeptide chip detection technology is used to detect blood samples from patients with COVID-19, cured patients with COVID-19 and uninfected healthy people. Comparative analysis is performed to obtain the immune characteristics of novel coronavirus-specific antibodies on the basis of the polypeptide chips and a corresponding analysis model. By means of a proactive experiment method, mouse serum samples are detected by means of polypeptide chip detection. 10 µL of mouse serum samples are used in the experiment, and preliminarily incubated and combined with the chips. Then, anti-mouse IgG antibodies and fluorescent antibodies are successively added for incubation and combination. After incubation is completed, the samples are loaded to an imager for fluorescence signal imaging, and a fluorescence intensity value of a characteristic is extracted. Original fluorescence intensity is normalized, to obtain a data matrix. Comparative analysis is performed with proactive experiment data, and whether the characteristics of polypeptide binding sites on the mouse serum and the polypeptide chip are the characteristics of the novel coronavirus-specific antibodies is identified.

### 2.2 Result analysis

a). Vaccine peptide-specific signal sequencing:
1. Two vaccine specific response modes are constructed according to vaccine peptide injection time series. Mode I: continuous rise over time (Pattern1); and Mode II: rise over time and maintaining stable after D13 (Pattern2).
2. A Spearman correlation coefficient is used to determine signals that conform to the above two modes, which is defined as A timing-sequence response peptide fragment set.
3. For each vaccine peptide, a polypeptide chip singal peptide fragment set B (vaccine-specific peptide fragment set) having sequence similarity to the vaccine peptide is calculated; and the sequence similarity is defined as the polypeptide chip signal peptide BLASTp being compared with a vaccine peptide design region, and Bit Score>=14, and a length of the intersection of the comparison region and the vaccine peptide exceeds 1/2 of a designed length of the vaccine peptide.
4. A Fisher exact test is performed on two polypeptide chip peptide fragment sets obtained in S2 and S3.
Results are shown in the table below.

**Table 6:**

| | Mode I | Mode II | #A timing-sequence response Peptide fragment set | #B vaccine specificity Peptide fragment set | Intersection of A and B | P value | Odds ratio |
|---|---|---|---|---|---|---|---|
| Combination 1 | 1095 | 629 | 1421 | 398 | 13 | 0.000698 | 2.982148 |
| Combination 2 | 758 | 428 | 991 | 364 | 8 | 0.006976 | 2.972366 |
| Combination 3 | 1377 | 732 | 1770 | 886 | 44 | 1.6E-14 | 4.289709 |
| Adjuvant control | 1488 | 796 | 1928 | 0 | 0 | 1 | 1 |

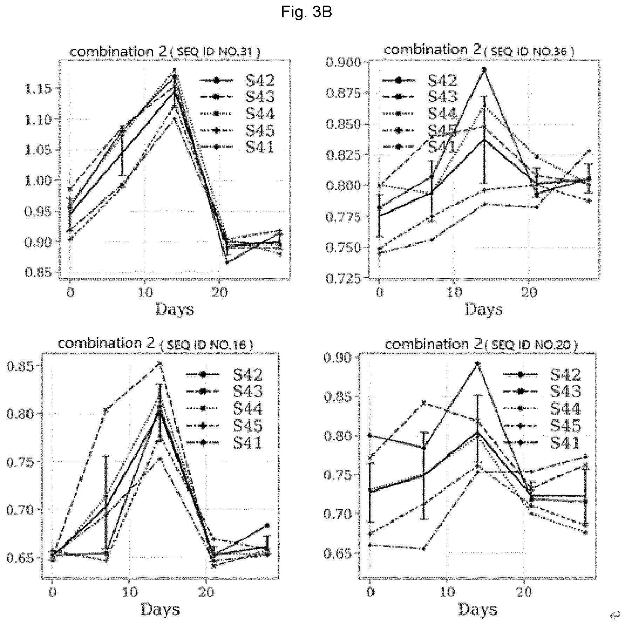

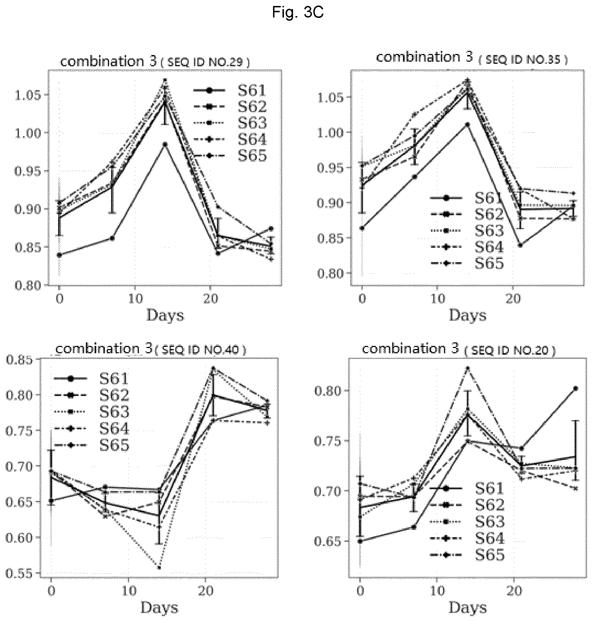

b). 95 percentile analysis method: For each group of mixed polypeptides, a polypeptide chip-specific signal of a single polypeptide is analyzed, and changes in specific signals for the single polypeptide from the single mouse in each group and within-group medians thereof are delineated in chronological order. A specific method includes the following.
1. An overall signal of a sequence similarity polypeptide chip peptide fragment of each vaccine peptide obtained in S3 of the method is extracted.
2. A 95 percentile of the overall signal at each time point is calculated.
3. A 95 percentile transition diagram of each mouse is plotted, and the within-group medians at each time point are calculated and plotted.
As shown in Fig. 3A to Fig. 3C (S+number in the figure represents the serial number of the experimental mice, that is, in each combination, the serial numbers of 5 mice immunized with each polypeptide), Fig. 3A shows antibody signals corresponding to 4 polypeptides in the combination 1. Fig. 3B shows antibody signals corresponding to 4 polypeptides in the combination 2. Fig. 3C shows antibody signals corresponding to 4 polypeptides in the combination 3. It may be seen that, All 3 combinations can stimulate the immunity of mice, so that antibody levels in a body can be improved, and the antibody signals corresponding to the polypeptide vaccine in the 3 combinations are all elevated to a certain extent at different time points.
c). Immunogenicity evaluation based on the polypeptide chip
Data distribution and characteristics thereof are analyzed based on a) and b), a polypeptide chip-based vaccine immunogenicity scoring system (referring to Table 7) is designed. An evaluation result is shown in Table 8.

**Table 7:**

| Item | Grouping | |
|---|---|---|
| | Scoring item | Scoring standard |
| 1) 95 percentile grouping number | A difference (A1) between a highest median and a DO median | [<=0.06, score=1; |
| | | 0.061-0.07, score=1.5-2; |
| | | 0.071-0.08, score=2.5-3; |
| | | 0.081-0.09, score=3-3.5; |
| | | 0.091-0.1, score=3.5-4; |
| | | 0.11-0.15, score=4.5; |
| | | >0.15, score=5] |
| | Whether the 95 percentile of the last sampling is increased compared with the end point (A2) | [Y=rise, |
| | | N=no rise; |
| | | Y=0.5, N=0] |
| | 95 percentile grouping number score (A) | Total score=5 |
| 2) Mouse performance | D0-D14 95 percentile rise in the number of mice (B1) | [30% of a score] |
| | D0-D21 95 percentile rise in the number of mice (B2) | [30% of a score] |
| | D14-D21 95 percentile rise in the number of mice (B3) | [20% of a score] |
| | D21-D28 95 percentile rise in the number of mice (B4) | [20% of a score] |
| | Mouse performance score (B) | Total score=5 |
| 3) Timing-sequence response and specificity | #A timing-sequence response peptide fragment set (C1) | |
| | #B vaccine specificity peptide fragment set (C2) | |
| | Intersection of A and B (C3) | |
| | Percentage of the intersection in set B (C4) | |
| | Timing-sequence response and specificity score (C) | [<=5, score=0.5; |
| | | 6-10, score=1; |
| | | 11-15, score=1.5; |
| | | 16-20, score=2; |
| | | 21-25, score=2.5; |
| | | 26-30, score=3; |
| | | 31-35, score=3.5; |
| | | 36-40, score=4; |
| | | 41-45, score=4.5; |
| | | >50, score=5] |
| Summary | Total single peptide | 95 percentile grouping number*3+mouse performance*2 |
| | | +timing-sequence response and specificity*1 |

**Table 8:**

| Item | Grouping | Combination 1 | | | | Combination 2 | | | | Combination 3 | | | |
|---|---|---|---|---|---|---|---|---|---|---|---|---|---|
| | Scoring item | lcx - 39 | icx - 33 | icx - 35 | lcx - 14 | icx - 13 | icx - 32 | icx - 23 | lcx - 16 | lcx - 21 | lcx - 31 | lcx - 14 | lcx - 16 |
| 1) 95 percentile grouping number | A1 | 0.1 | 0.05 | 0.21 | 0.1 | 0.4 | 0.1 | 0.2 | 0.1 | 0.2 | 0.1 | 0.1 | 0.1 |
| | A2 | N | N | N | N | N | Y | Y | N | N | N | N | Y |
| | A | 4.5 | 1 | 5 | 3 | 5 | 1.5 | 5 | 3 | 4.5 | 4.5 | 4.5 | 4 |
| 2) Mouse performance | B1 | 5 | 5 | 5 | 1 | 5 | 5 | 5 | 5 | 5 | 5 | 0 | 5 |
| | B2 | 0 | 5 | 0 | 5 | 0 | 5 | 1 | 2 | 0 | 0 | 5 | 5 |
| | B3 | 0 | 1 | 0 | 5 | 0 | 0 | 0 | 2 | 0 | 0 | 5 | 0 |
| | B4 | 1 | 2 | 1 | 1 | 1 | 2 | 2 | 2 | 1 | 1 | 1 | 2 |
| | B | 1.7 | 3.6 | 1.7 | 3 | 1.7 | 3.4 | 2.2 | 2.9 | 1.9 | 1.9 | 1.9 | 3.8 |
| 3) Timing-sequence response and specificity | C1 | 1421 | | | | 991 | | | | 1770 | | | |
| | C2 | 398 | | | | 364 | | | | | | | |
| | C3 | 13 | | | | g | | | | 44 | | | |
| | C4 | 3.266331658 | | | | 2.197802198 | | | | 4.966139955 | | | |
| | C | 0.5 | | | | 0.5 | | | | 0.5 | | | |
| Total single peptide | | 17 | 10.7 | 18.9 | 16 | 18.9 | 11.8 | 19.9 | 15.3 | 17.8 | 17.8 | 17.8 | 20.1 |
| Total in grouping | | 625 | | | | 65.9 | | | | 73.5 | | | |

### 3. In-vitro live virus neutralization experiment with mouse serum

3.1 This embodiment is consistent with the previous embodiment. 10-Fold dilution at a dilution of 10⁻¹-10⁻¹⁰ is continuously performed on the live coronavirus, the diluted virus is separately inoculated to a 96-well culture plate, and a column of 8 wells are inoculated for each dilution. After cell suspension is added to each well for 5 days of co-culture, the number of holes with Cytopathic Effect (CPE) is counted under a microscope, and an infective dose TCID50 of virus half cell cultures is obtained.

Complement inactivation is performed on the mouse serum at 56°C for 30 min. 1:10, 1:20, 1:40, 1:80, 1:160, 1:320, 1:640, and 1:1280 gradient dilution is performed on the mouse serum after inactivation. A solution containing 100 TCID50 viruses and the serum of each dilution are equivalently mixed, and then incubated in a 37°C water bath for 1 h. The incubated virus serum mixed solution is added to the 96-well culture plate pre-inoculated with vero cells, and then the culture plate is incubated in an environment of 37°C and 5%CO₂. After inoculation, CPE is observed every day, and a final result is determined at Day 7.

### 3.2 Experimental result

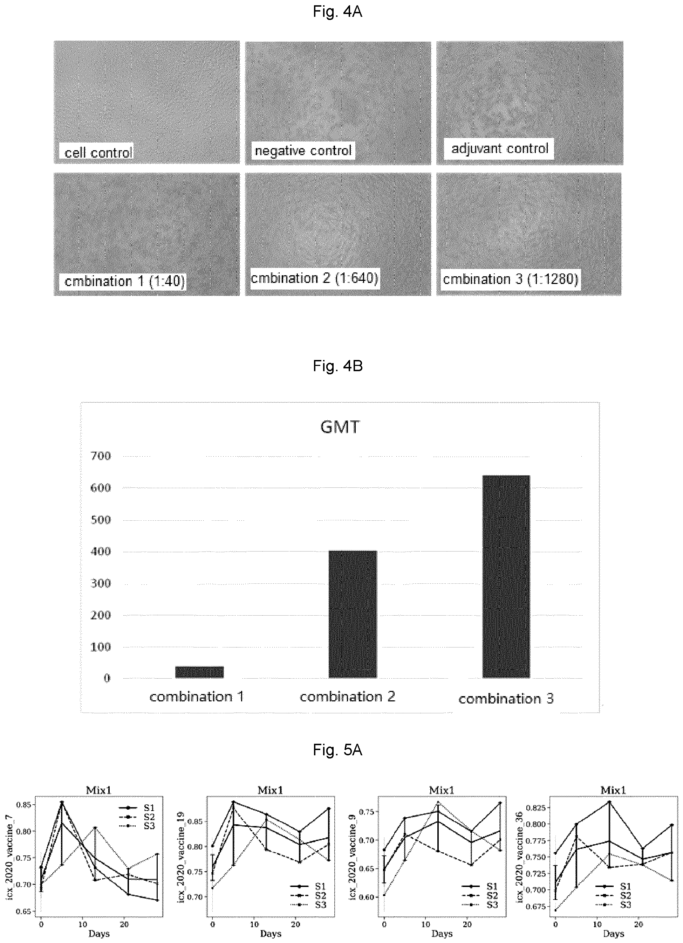

As shown in Fig. 4A, 4B and the table below, the serum obtained from the mice immunized with polypeptides can have neutralizing activity to the novel coronavirus. Antibodies in mice injected with combination 2 and combination 3 have a better neutralization effect. 75% of the mice injected with the polypeptide combination 2 produce neutralizing antibodies, the highest neutralizing titer reaches 1:640, and the geometric mean neutralizing titer is 1:403. 50% of the mice injected with the polypeptide combination 3 produce neutralizing antibodies, the highest neutralizing titer reaches 1:1280, and the geometric mean neutralizing titer is 1:640. A trend between neutralizing effect groups is consistent with the scores in Table 8 of 2.2 c). Results show that, the novel coronavirus vaccine peptides designed with the aid of the polypeptide chips can neutralize the novel coronavirus.

**Table 9:**

| Combination | Neutralizing antibody positive rate (%) | Highest neutralizing titer | Geometric mean neutralizing titer (GMT) |
|---|---|---|---|
| Combination 1 | 25 | 1:40 | 1:40 |
| Combination 2 | 75 | 1:640 | 1:403 |
| Combination 3 | 50 | 1:1280 | 1:640 |

### Embodiment III Effectiveness verification of polypeptide-coupled protein vaccine

### I. Experimental operation

### 1. Preparation of polypeptide-coupled KLH

Keyhole Limpet Hemocyanin (KLH) is a free blue respiratory pigment found in the hemolymph of mollusks and arthropods (such as spiders and beetles), and has high immunogenicity, which is the most commonly used carrier protein. 40 polypeptides are selected from Table 10 below to form 10 polypeptide combinations, and Mix4 and Mix9 are consistent with combination 1 and combination 2 in Embodiment II, respectively. Each group of polypeptides are respectively coupled to the KLH, and steps of a polypeptide-KLH coupling experiment include the following.
1) 0.1M-MES and 0.5M-NaCl are used to prepare a reaction buffer (pH 6.0), KLH is diluted to 1mg/mL with the reaction buffer, and take 1 mL of the mixture for later use.
2) EDC (0.4 mg) with a final concentration being 2 mM and 5mM sulfo-NHS (1.1 mg) are added to the solution in 1.
3) Reaction is performed at room temperature for 15 min after well mixing.
4) β-mercaptoethanol (1.4ul) with the final concentration being 20 mM is added to the reaction solution to end the reaction of EDC, and incubation is performed at room temperature for 10 min.
5) Hapten (polypeptide) in PBS is added to an activated KLH protein solution, and reaction is performed at room temperature for 2h.
6) Hydroxylamine with the final concentration being 10 mM or 20-50mM Tris or lysine is added to end the reaction.

### 2. Immunized mice

In an experiment, 5 to 6-week-old female Balb/c mice are randomly divided into 12 groups, 10 of which are polypeptide-KLH experimental groups (combinations 1-10), 1 is an individual KLH group with polypeptides uncoupled, and 1 is a simple adjuvant group. Each group has 5 mice.

40 bars of polypeptide powder are respectively dissolved and diluted to a polypeptide solution with a final concentration being 2 mg/ml by using a PBS solution. For the polypeptide-KLH experimental group, according to grouping information, and in each group of combinations, 50 µg of each polypeptide is mixed to form a mixed solution containing a total of 200 µg of polypeptides as a polypeptide solution for first injection in mice. Then, in each group of combinations, 25 µg of each polypeptide is mixed to form a 100 µg of the mixed solution as the polypeptide solution for second and third injections in mice. First, second and third injections are respectively and correspondingly injected to the mice in each group according to grouping at Day 0, 7 and 14. During injection, the polypeptide solution and the equal volume of adjuvant Imject Alum Adjuvant (Thermo Fisher Scientific) are mixed, and then are subcutaneously administrated to gastrocnemius muscle of two legs of mice by using a microsyringe. In the individual KLH control group, the same amount of KLH as in the polypeptide-KLH group is used to be mixed with an equal volume of Imject Alum Adjuvant for injection. In the simple adjuvant group, only the Imject Alumn original concentration solution having a same volume as a final solution of the polypeptide experimental group is used for injection. The individual KLH control group and the simple adjuvant group are administered for experiment in the same manner and frequency as the polypeptide experimental group.

Before the mice in each experimental group are immunized (Day 0) and at Days 7, 14, 21 after initial immunization (Days 7 and 14 are before injection), mouse tail vein blood samples are collected. The blood sample volumes collected at each time point are about 100-200 µL. Serum is prepared through separation for polypeptide chip detection. Day 28 after the mice are immunized is used as an experimental end point. The mice are killed. Then, blood is collected and separated to prepare supernatant for neutralization experiments.

**Table 10:**

| Grouping | Polypeptide 1 | Polypeptide 2 | Polypeptide 3 | Polypeptide 4 |
|---|---|---|---|---|
| Mix1 | icx_7 | icx_19 | icx_9 | icx_36 |
| Mix2 | icx_34 | icx_22 | icx_11 | icx_10 |
| Mix3 | icx_17 | icx_2 | icx_6 | icx_29 |
| Mix4 | icx_39 | icx_33 | icx_35 | icx_14 |
| Mix5 | icx_8 | icx_18 | icx_27 | icx_37 |
| Mix6 | icx_30 | icx_5 | icx_4 | icx_1 |
| Mix7 | icx_38 | icx_25 | icx_15 | icx_21 |
| Mix8 | icx_26 | icx_31 | icx_28 | icx_24 |
| Mix9 | icx_3 | icx_32 | icx_23 | icx_16 |
| Mix10 | icx_13 | icx_40 | icx_12 | icx_20 |

### 2. Detection of peptide immunogenicity by polypeptide chip

### 2.1 Experimental operation

In this embodiment, the serum of the immunized mice is detected by means of the same polypeptide chip detection method in Embodiment II.

### 2.1 Experimental operation

### 2.2 Result analysis

a). Vaccine peptide-specific signal sequencing: A method is consistent with that in Embodiment II. Results are shown in the table below.

**Table 11:**

| | Mode I | Mode II | #A timing-sequence response peptide fragment set | #B vaccine specificity peptide fragment set | Intersection of A and B | P value | Odds ratio |
|---|---|---|---|---|---|---|---|
| Mix1 | 1189 | 90 | 1212 | 389 | 1 | 0.2780509 | 0.276579552 |
| Mix2 | 93 | 45 | 133 | 431 | 1 | 0.3543766 | 2.303417386 |
| Mix3 | 1437 | 351 | 1494 | 190 | 2 | 1 | 0.912065816 |
| Mix4 | 806 | 108 | 823 | 398 | 7 | 0.0165432 | 2.741062428 |
| Mix5 | 145 | 125 | 230 | 251 | 1 | 0.363429 | 2.228571429 |
| Mix6 | 77 | 243 | 288 | 268 | 4 | 0.0031902 | 6.875275088 |
| Mix7 | 85 | 196 | 257 | 682 | 3 | 0.1567622 | 2.222863192 |
| Mix8 | 162 | 255 | 352 | 541 | 1 | 1 | 0.692224388 |
| Mix9 | 100 | 458 | 511 | 364 | 1 | 1 | 0.704362321 |
| Mix10 | 67 | 90 | 145 | 529 | 1 | 0.4441511 | 1.713575977 |
| Adjuvant control | 37 | 32 | 62 | 0 | 0 | 1 | |
| KLH control | 846 | 127 | 926 | 0 | 0 | 1 | |

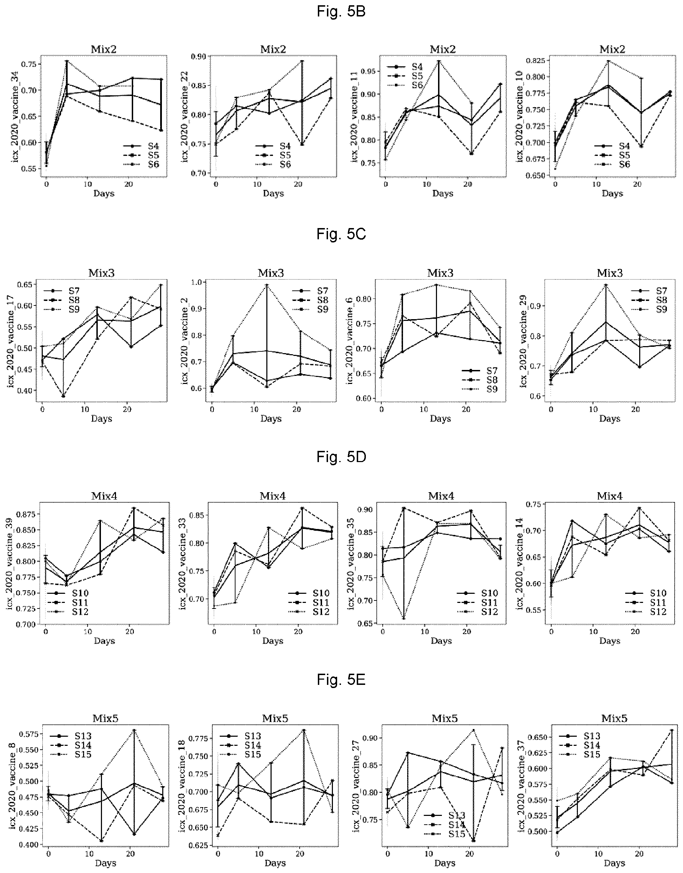

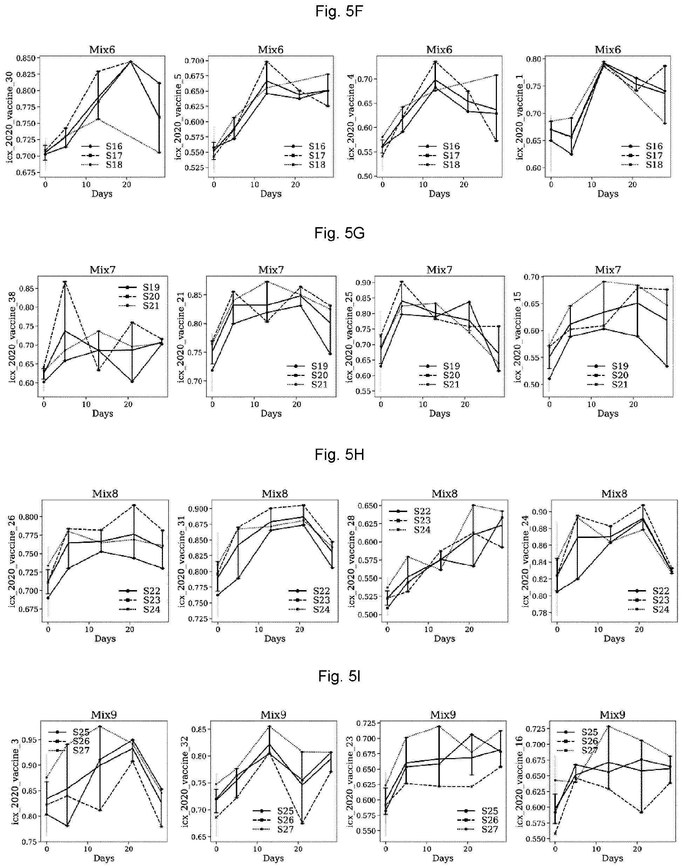

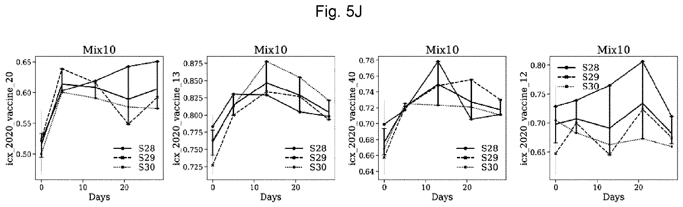

b). 95 percentile analysis method: The method is consistent with that in Embodiment II.
As shown in Fig. 5A to Fig. 5J (S+number in the figure represents the serial number of the experimental mice, that is, in each combination, the serial numbers of 3 mice immunized with each polypeptide), Fig. 5A to Fig. 5J show antibody signals corresponding to 4 polypeptides in each mix. It may be seen that, All 10 combinations can stimulate the immunity of mice, so that antibody levels in a body can be improved, and the antibody signals corresponding to the polypeptide vaccine in the 10 combinations are all elevated to a certain extent at different time points.
c). Immunogenicity evaluation based on the polypeptide chip
10 combinations are evaluated by the polypeptide chip-based vaccine immunogenicity scoring system. An evaluation method is consistent with that in Embodiment II. Evaluation results are show in the table below.

**Table 12:**

| | | 1) 95 percentile grouping number | | | 2) Mouse performance | | | | | 3) Timing-sequence response and specificity | | | | | Total single peptide | Total in grouping |
|---|---|---|---|---|---|---|---|---|---|---|---|---|---|---|---|---|
| grouping | Scoring item | A1 | A2 | A | B1 | B2 | B3 | B4 | B | C1 | C2 | C3 | C4 | C | | |
| MIX1 | icx_7 | 0.11 | N | 4 | 1 | 2 | 0 | 1 | 0.9 | 1212 | 389 | 1 | 0.257 | 0.5 | 14.3 | 55.3 |
| | icx_19 | 0.09 | Y | 4 | 3 | 3 | 0 | 2 | 1.9 | 1212 | 389 | 1 | 0.257 | 0.5 | 16.3 | |
| | icx_9 | 0.08 | Y | 3.5 | 3 | 3 | 0 | 2 | 1.9 | 1212 | 389 | 1 | 0.257 | 0.5 | 14.8 | |
| | icx_36 | 0.06 | Y | 2 | 3 | 2 | 0 | 2 | 1.7 | 1212 | 389 | 1 | 0.257 | 0.5 | 9.9 | |
| MIX2 | icx_34 | 0.14 | N | 4.5 | 3 | 3 | 1 | 0 | 1.7 | 133 | 431 | 1 | 0.232 | 0.5 | 17.4 | 70.8 |
| | icx_22 | 0.09 | Y | 4 | 3 | 2 | 2 | 3 | 2.3 | 133 | 431 | 1 | 0.232 | 0.5 | 17.1 | |
| | icx_11 | 0.11 | Y | 5 | 3 | 2 | 0 | 2 | 1.7 | 133 | 431 | 1 | 0.232 | 0.5 | 18.9 | |
| | icx_10 | 0.10 | Y | 4.5 | 3 | 2 | 0 | 2 | 1.7 | 133 | 431 | 1 | 0.232 | 0.5 | 17.4 | |
| MIX3 | icx_17 | 0.12 | Y | 5 | 3 | 3 | 1 | 2 | 2.1 | 1494 | 190 | 2 | 1.053 | 1 | 20.2 | 75.2 |
| | icx_2 | 0.15 | N | 4.5 | 2 | 3 | 2 | 0 | 1.6 | 1494 | 190 | 2 | 1.053 | 1 | 17.7 | |
| | icx_6 | 0.11 | N | 4.5 | 3 | 3 | 1 | 0 | 1.7 | 1494 | 190 | 2 | 1.053 | 1 | 17.9 | |
| | icx_29 | 0.20 | N | 5 | 3 | 3 | 0 | 1 | 1.7 | 1494 | 190 | 2 | 1.053 | 1 | 19.4 | |
| MIX4 | icx_39 | 0.06 | N | 1.5 | 2 | 3 | 2 | 1 | 1.8 | 823 | 398 | 7 | 1.759 | 1 | 9.1 | 59.5 |
| | icx_33 | 0.13 | N | 4.5 | 3 | 3 | 2 | 1 | 2.1 | 823 | 398 | 7 | 1.759 | 1 | 18.7 | |
| | icx_35 | 0.08 | N | 3 | 3 | 3 | 1 | 0 | 1.7 | 823 | 398 | 7 | 1.759 | 1 | 13.4 | |
| | icx_14 | 0.11 | N | 4.5 | 3 | 3 | 2 | 0 | 1.9 | 823 | 398 | 7 | 1.759 | 1 | 18.3 | |
| MIX5 | icx_8 | 0.02 | N | 1 | 2 | 2 | 2 | 1 | 1.6 | 230 | 251 | 1 | 0.398 | 0.5 | 6.7 | 44.5 |
| | icx_18 | 0.03 | N | 1 | 2 | 3 | 2 | 1 | 1.8 | 230 | 251 | 1 | 0.398 | 0.5 | 7.1 | |
| | icx_27 | 0.09 | Y | 4 | 3 | 2 | 1 | 1 | 1.7 | 230 | 251 | 1 | 0.398 | 0.5 | 15.9 | |
| | icx_37 | 0.08 | Y | 3.5 | 3 | 3 | 1 | 1 | 1.9 | 230 | 251 | 1 | 0.398 | 0.5 | 14.8 | |
| MIX6 | icx_30 | 0.14 | N | 4.5 | 3 | 3 | 2 | 0 | 1.9 | 288 | 268 | 4 | 1.493 | 1 | 18.3 | 74.7 |
| | icx_5 | 0.12 | Y | 5 | 3 | 3 | 1 | 2 | 2.1 | 288 | 268 | 4 | 1.493 | 1 | 20.2 | |
| | icx_4 | 0.13 | N | 4.5 | 3 | 3 | 1 | 1 | 1.9 | 288 | 268 | 4 | 1.493 | 1 | 18.3 | |
| | icx_1 | 0.12 | N | 4.5 | 3 | 3 | 0 | 1 | 1.7 | 288 | 268 | 4 | 1.493 | 1 | 17.9 | |
| MIX7 | icx_38 | 0.11 | Y | 5 | 2 | 2 | 1 | 1 | 1.4 | 257 | 682 | 3 | 0.440 | 0.5 | 18.3 | 67.9 |
| | icx_21 | 0.10 | N | 4 | 3 | 3 | 2 | 0 | 1.9 | 257 | 682 | 3 | 0.440 | 0.5 | 16.3 | |
| | icx_25 | 0.15 | N | 4.5 | 3 | 3 | 1 | 0 | 1.7 | 257 | 682 | 3 | 0.440 | 0.5 | 17.4 | |
| | icx_15 | 0.10 | N | 4 | 3 | 3 | 1 | 0 | 1.7 | 257 | 682 | 3 | 0.440 | 0.5 | 15.9 | |
| MIX8 | icx_26 | 0.06 | N | 1 | 3 | 3 | 1 | 0 | 1.7 | 352 | 541 | 1 | 0.185 | 0.5 | 6.9 | 52.1 |
| | icx_31 | 0.10 | N | 4 | 3 | 3 | 2 | 0 | 1.9 | 352 | 541 | 1 | 0.185 | 0.5 | 16.3 | |
| | icx_28 | 0.10 | Y | 4.5 | 3 | 3 | 2 | 1 | 2.1 | 352 | 541 | 1 | 0.185 | 0.5 | 18.2 | |
| | icx_24 | 0.07 | N | 2 | 3 | 3 | 3 | 0 | 2.1 | 352 | 541 | 1 | 0.185 | 0.5 | 10.7 | |
| MIX9 | icx_3 | 0.93 | N | 3.5 | 2 | 3 | 2 | 0 | 1.6 | 511 | 364 | 1 | 0.275 | 0.5 | 14.2 | 61.6 |
| | icx_32 | 0.10 | Y | 4.5 | 3 | 2 | 0 | 2 | 1.7 | 511 | 364 | 1 | 0.275 | 0.5 | 17.4 | |
| | icx_23 | 0.08 | Y | 3.5 | 3 | 3 | 1 | 2 | 2.1 | 511 | 364 | 1 | 0.275 | 0.5 | 15.2 | |
| | icx_16 | 0.08 | Y | 3.5 | 3 | 3 | 1 | 1 | 1.9 | 511 | 364 | 1 | 0.275 | 0.5 | 14.8 | |
| MIX10 | icx_20 | 0.10 | Y | 4.5 | 3 | 3 | 1 | 2 | 2.1 | 145 | 529 | 1 | 0.189 | 0.5 | 18.2 | 48.2 |
| | icx_13 | 0.09 | N | 3.5 | 3 | 3 | 0 | 0 | 1.5 | 145 | 529 | 1 | 0.189 | 0.5 | 14 | |
| | icx_40 | 0.07 | N | 2 | 3 | 3 | 1 | 1 | 1.9 | 145 | 529 | 1 | 0.189 | 0.5 | 10.3 | |
| | icx_12 | 0.03 | N | 1 | 1 | 2 | 2 | 0 | 1.1 | 145 | 529 | 1 | 0.189 | 0.5 | 5.7 | |

Sorting is performed according to the total scores of the above groups, and sorting results are shown in the table below.

**Table 13:**

| Ranking | Combination | Total in grouping |
|---|---|---|
| 1 | Mix3 | 75.2 |
| 2 | Mix6 | 74.7 |
| 3 | Mix2 | 70.8 |
| 4 | Mix7 | 67.9 |
| 5 | Mix9 | 61.6 |
| 6 | Mix4 | 59.5 |
| 7 | Mix1 | 55.3 |
| 8 | Mix8 | 52.1 |
| 9 | Mix10 | 48.2 |
| 10 | Mix5 | 44.5 |

Since Mix4 and Mix9 are consistent with combination 1 and combination 2 in Embodiment II, respectively, it indicates that Mix4 and Mix9 have neutralizing effects according to the results in Embodiment II. Based on the ranking of Mix3, Mix6, Mix2, and Mix7 higher than Mix4 and Mix9, it is speculated that Mix3, Mix6, Mix2, and Mix7 also have potential effectiveness, and may be used for vaccine development.

### Embodiment IV Effectiveness verification of compatibility of polypeptide vaccine and adjuvant

### I. Experimental operation

### 1. Immunized mice

ICX ID:icx_16(SEQ ID NO:20), 21(SEQ ID NO:29), 24(SEQ ID NO:22), 32(SEQ ID NO:36), 33(SEQ ID NO:3), 35(SEQ ID NO:34), and 37(SEQ ID NO:21) are selected to be combined into 7 peptides, to perform screening and verification of compatibility effectiveness with different adjuvants. In this experiment, 6 adjuvants are used for screening, which respectively are AddaVax (also recorded as MF59, InvivoGen), Imject Alumn (Thermo Scientific), Alhydrogel (InvivoGen), Adju-Phos (InvivoGen), Novavax (also recorded as MA103A, Maxvax), and MA103B (also recorded as positively charged, Maxvax). In an experiment, 5 to 6-week-old female Balb/c mice are randomly divided into 12 groups, 6 of which are experimental groups combining 7 peptides with different adjuvants, and 6 are simple adjuvant groups. Each group has 5 mice.

7 bars of polypeptide powder are respectively dissolved and diluted to a polypeptide solution with a final concentration being 2 mg/ml by using a PBS solution. For the 7-peptide combination experimental group, according to grouping information, and in each group of combinations, 30 µg of each polypeptide is mixed to form a mixed solution containing a total of 210 µg of polypeptides as polypeptide solutions for first, second and third injections in mice. First, second and third injections are respectively and correspondingly injected to the mice in each group according to the grouping at Day 0, 7 and 14. During injection, the polypeptide solution and the equal volume of corresponding adjuvant are mixed, and then are subcutaneously administrated to gastrocnemius muscle of two legs of mice by using a microsyringe. In the simple adjuvant group, the PBS is used instead of the 7-peptide mixed solution to mix with the equal volume of the corresponding adjuvant solution for injection. The simple adjuvant group is administered for experiment in the same manner and frequency as the polypeptide experimental group.

Before the mice in each experimental group are immunized (Day 0) and at Days 7 and 14 after initial immunization (Days 7 and 14 are before injection), mouse tail vein blood samples are collected. The blood sample volumes collected at each time point are about 100-200 µL. Serum is prepared through separation for polypeptide chip detection. Day 21 after the mice are immunized is used as an experimental end point. The mice are killed. Then, blood is collected and separated to prepare supernatant for neutralization experiments.

### 2. Detection of peptide immunogenicity by polypeptide chip

### 2.1 Experimental operation

In this embodiment, the serum of the immunized mice is detected by means of the same polypeptide chip detection method in Embodiment II.

### 2.2 Result analysis

a). Vaccine peptide-specific signal sequencing: being consistent with that in Embodiment II. Results are shown in the table below.

**Table 14:**

| Mix | Immunogen | Adjuvant | Mode I | Mode II | #A timing-sequence response peptide fragment set | #B vaccine specificity peptide fragment set | Intersection of A and B | P value | Odds ratio |
|---|---|---|---|---|---|---|---|---|---|
| 1 | 7-peptide mixed solution | AddaVax | 87 | 157 | 237 | 1244 | 0 | 0.27371 | 0 |
| 2 | 7-peptide mixed solution | Imject Alumn | 74 | 31 | 100 | 1244 | 3 | 0.0496 | 3.74734 |
| 3 | 7-peptide mixed solution | Alhydroge l | 73 | 53 | 123 | 1244 | 2 | 0.26755 | 2.00038 |
| 4 | 7-peptide mixed solution | Adju-Phos | 23 | 183 | 203 | 1244 | 0 | 0.42079 | 0 |
| 5 | 7-peptide mixed solution | MA103A | 102 | 171 | 260 | 1244 | 3 | 0.4766 | 1.41255 |
| 6 | 7-peptide mixed solution | MA103B | 41 | 115 | 153 | 1244 | 0 | 0.64098 | 0 |
| 7 | PBS | AddaVax | 180 | 311 | 474 | 0 | 0 | 1 | NA |
| 8 | PBS | Imject Alumn | 889 | 544 | 1341 | 0 | 0 | 1 | NA |
| 9 | PBS | Alhydroge l | 251 | 373 | 586 | 0 | 0 | 1 | NA |
| 10 | PBS | Adju-Phos | 113 | 82 | 181 | 0 | 0 | 1 | NA |
| 11 | PBS | MA103A | 521 | 806 | 1176 | 0 | 0 | 1 | NA |
| 12 | PBS | MA103B | 128 | 98 | 217 | 0 | 0 | 1 | NA |

| | | | | | | | | | |
|---|---|---|---|---|---|---|---|---|---|
| NA in the table above represents: not applicable. | | | | | | | | | |

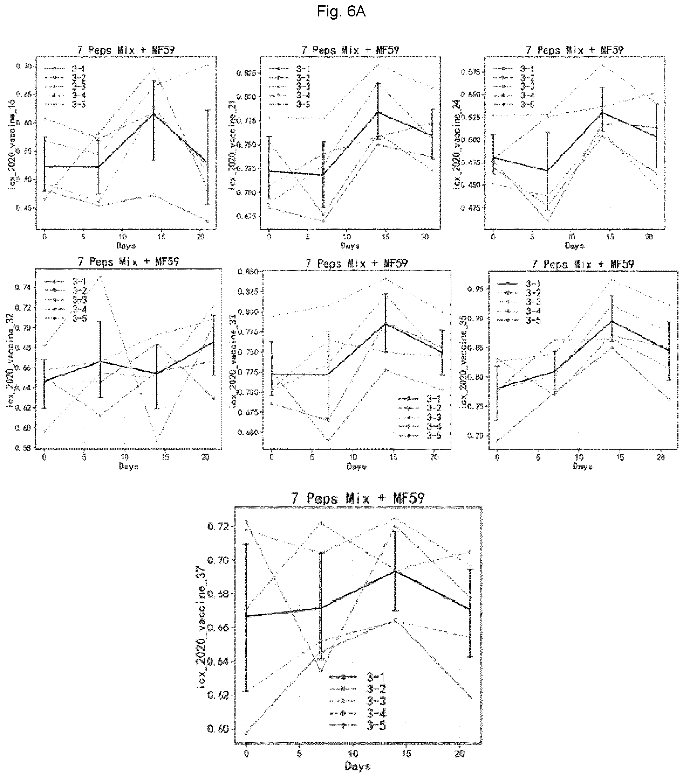

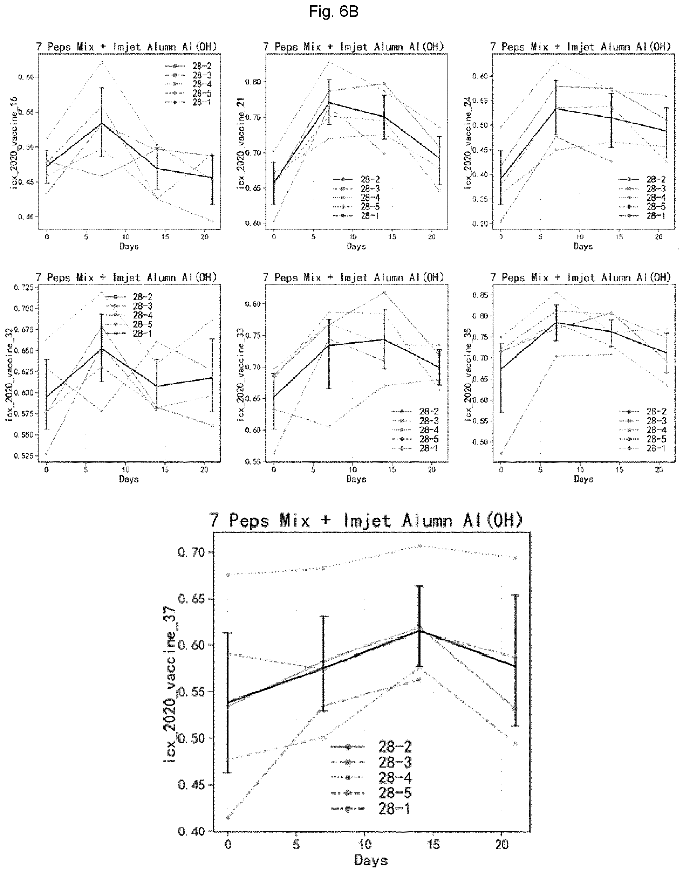

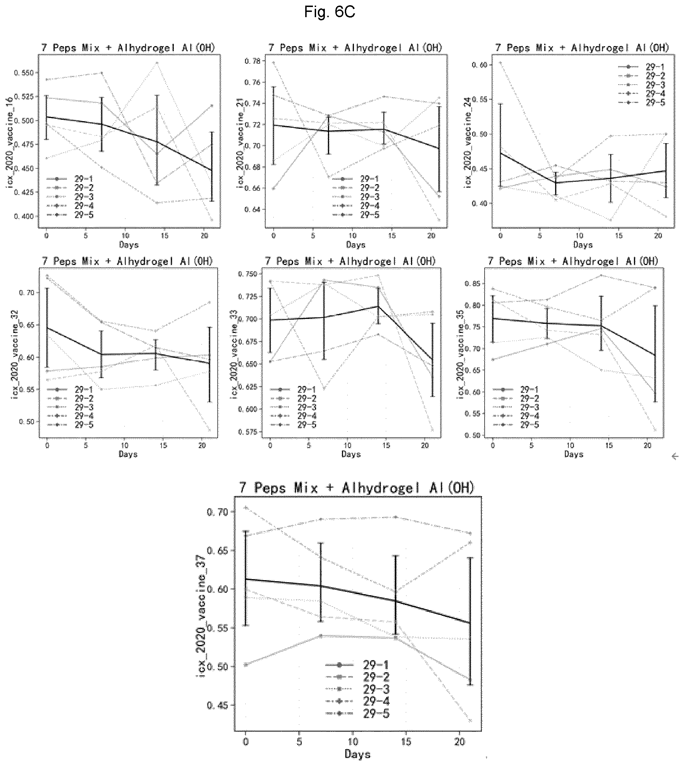

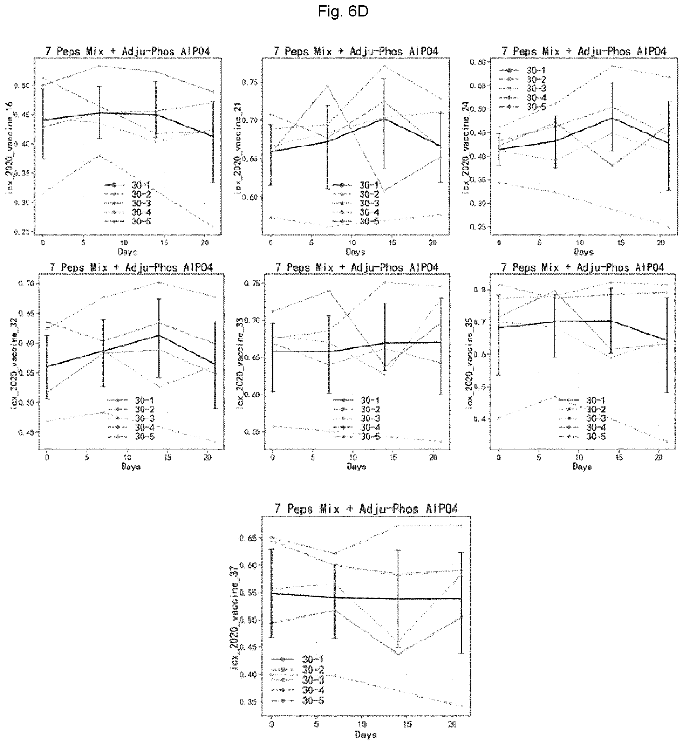

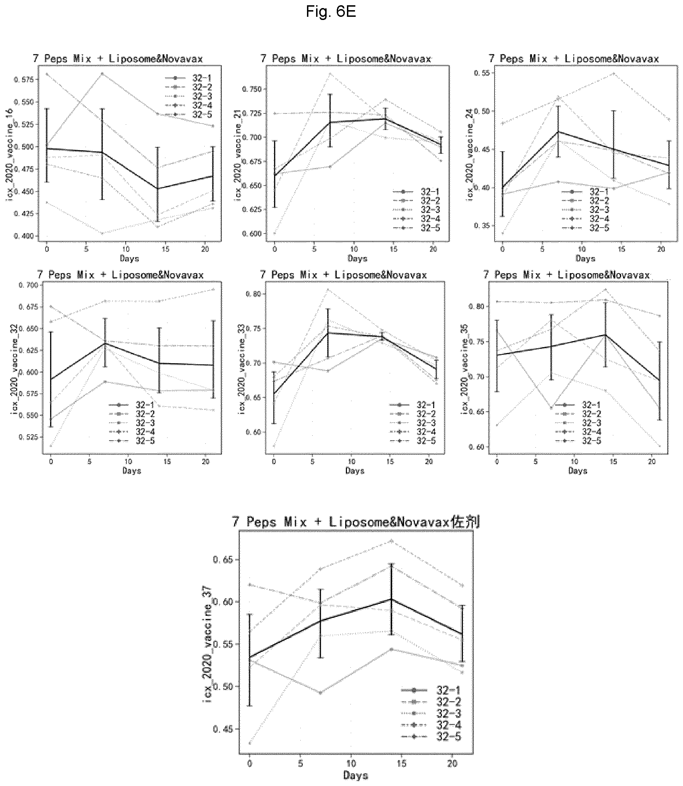

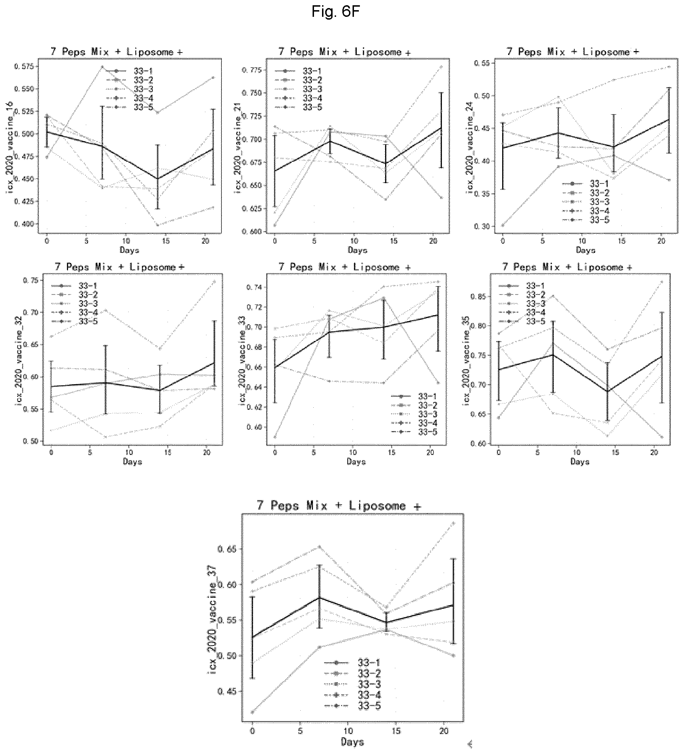

b). 95 percentile analysis method: The method is consistent with that in Embodiment II. Results are shown in Fig. 6A to Fig. 6F. Figures show antibody production at different time points after 7 peptides are co-immunized with each adjuvant in mice. The 7 peptides are successively shown according to an order of icx_16, 21, 24, 32, 33, 35, and 37. The order of the adjuvants corresponding to Fig. 6A to Fig. 6F is the same as the order of the adjuvants of 1 to 6 in the table above.
c). Immunogenicity evaluation based on the polypeptide chip This embodiment is consistent with Embodiment II. Each combination is evaluated by the polypeptide chip-based vaccine immunogenicity scoring system. Evaluation results are show in the table below.

**Table 15:**

| | | 1) 95 percentile grouping number | | | 2) Mouse performance | | | | 3) Timing-sequence response and specificity | | | | | | |
|---|---|---|---|---|---|---|---|---|---|---|---|---|---|---|---|
| Groupi ng | Scoring item | A1 | A2 | A | B 1 | B 2 | B 3 | B | C1 | C2 | C3 | C4 | C | Total singl e pepti de | Total in group ing |
| AddaV ax | icx_16 | 0 | N | 0 | 1 | 0 | 3 | 1.5 | 237 | 1244 | 0 | 0.000 | 0 | 3 | 105 |
| | icx_21 | 0.055 | Y | 4 | 3 | 5 | 4 | 3.5 | 237 | 1244 | 0 | 0.000 | 0 | 19 | |
| | icx_24 | 0.05 | Y | 4 | 3 | 4 | 3 | 2.9 | 237 | 1244 | 0 | 0.000 | 0 | 17.8 | |
| | icx_32 | 0.04 | Y | 3.5 | 3 | 4 | 2 | 2.5 | 237 | 1244 | 0 | 0.000 | 0 | 15.5 | |
| | icx_33 | 0.05 | Y | 4 | 3 | 5 | 4 | 3.5 | 237 | 1244 | 0 | 0.000 | 0 | 19 | |
| | icx_35 | 0.05 | N | 3.5 | 4 | 4 | 1 | 2.4 | 237 | 1244 | 0 | 0.000 | 0 | 15.3 | |
| | icx_37 | 0.055 | N | 3.5 | 0 | 3 | 5 | 2.6 | 237 | 1244 | 0 | 0.000 | 0 | 15.7 | |
| Imjet Alumn | icx_16 | 0 | N | 0 | 1 | 1 | 3 | 1.7 | 100 | 1244 | 3 | 0.241 | 1 | 3.9 | 115 |
| | icx_21 | 0.08 | N | 5 | 5 | 3 | 1 | 2.5 | 100 | 1244 | 3 | 0.241 | 1 | 20.5 | |
| | icx_24 | 0.145 | N | 5 | 5 | 4 | 0 | 2.3 | 100 | 1244 | 3 | 0.241 | 1 | 20.1 | |
| | icx_32 | 0.065 | N | 4.5 | 5 | 3 | 1 | 2.5 | 100 | 1244 | 3 | 0.241 | 1 | 19 | |
| | icx_33 | 0.09 | N | 5 | 5 | 4 | 1 | 2.7 | 100 | 1244 | 3 | 0.241 | 1 | 20.9 | |
| | icx_35 | 0.11 | N | 5 | 5 | 3 | 0 | 2.1 | 100 | 1244 | 3 | 0.241 | 1 | 19.7 | |
| | icx_37 | 0.025 | Y | 2 | 0 | 4 | 4 | 2.4 | 100 | 1244 | 3 | 0.241 | 1 | 11.3 | |
| Alhydr ogel | icx_16 | 0 | N | 0 | 2 | 1 | 2 | 1.6 | 123 | 1244 | 2 | 0.161 | 1 | 3.7 | 66.8 |
| | icx_21 | 0.04 | N | 3 | 5 | 2 | 0 | 1.9 | 123 | 1244 | 2 | 0.161 | 1 | 13.3 | |
| | icx_24 | 0.03 | N | 2 | 3 | 2 | 1 | 1.7 | 123 | 1244 | 2 | 0.161 | 1 | 9.9 | |
| | icx_32 | 0 | N | 0 | 3 | 1 | 2 | 1.9 | 123 | 1244 | 2 | 0.161 | 1 | 4.3 | |
| | icx_33 | 0.012 | N | 1 | 2 | 1 | 0 | 0.8 | 123 | 1244 | 2 | 0.161 | 1 | 5.1 | |
| | icx_35 | 0.06 | Y | 5 | 4 | 3 | 2 | 2.6 | 123 | 1244 | 2 | 0.161 | 1 | 20.7 | |
| | icx_37 | 0.025 | N | 1.5 | 4 | 2 | 2 | 2.4 | 123 | 1244 | 2 | 0.161 | 1 | 9.8 | |
| Adju-P hos | icx_16 | 0.025 | N | 1.5 | 2 | 2 | 1 | 1.4 | 203 | 1244 | 0 | 0.000 | 0 | 7.3 | 109 |
| | icx_21 | 0.055 | N | 3.5 | 3 | 3 | 1 | 1.9 | 203 | 1244 | 0 | 0.000 | 0 | 14.3 | |
| | icx_24 | 0.075 | N | 5 | 3 | 4 | 1 | 2.1 | 203 | 1244 | 0 | 0.000 | 0 | 19.2 | |
| | icx_32 | 0.05 | N | 3.5 | 2 | 2 | 2 | 1.8 | 203 | 1244 | 0 | 0.000 | 0 | 14.1 | |
| | icx_33 | 0.05 | N | 3.5 | 4 | 5 | 2 | 3 | 203 | 1244 | 0 | 0.000 | 0 | 16.5 | |
| | icx_35 | 0.08 | N | 5 | 3 | 4 | 2 | 2.5 | 203 | 1244 | 0 | 0.000 | 0 | 20 | |
| | icx_37 | 0.05 | Y | 4 | 3 | 4 | 3 | 2.9 | 203 | 1244 | 0 | 0.000 | 0 | 17.8 | |
| MA103 A | icx_16 | 0.015 | Y | 1.5 | 2 | 3 | 3 | 2.4 | 260 | 1244 | 3 | 0.241 | 1 | 9.8 | 127 |
| | icx_21 | 0.08 | N | 5 | 5 | 5 | 1 | 2.9 | 260 | 1244 | 3 | 0.241 | 1 | 21.3 | |
| | icx_24 | 0.11 | N | 5 | 5 | 5 | 0 | 2.5 | 260 | 1244 | 3 | 0.241 | 1 | 20.5 | |
| | icx_32 | 0.065 | N | 4.5 | 4 | 4 | 1 | 2.4 | 260 | 1244 | 3 | 0.241 | 1 | 18.8 | |
| | icx_33 | 0.08 | N | 5 | 5 | 5 | 1 | 2.9 | 260 | 1244 | 3 | 0.241 | 1 | 21.3 | |
| | icx_35 | 0.115 | N | 5 | 5 | 5 | 0 | 2.5 | 260 | 1244 | 3 | 0.241 | 1 | 20.5 | |
| | icx_37 | 0.042 | N | 3 | 4 | 3 | 2 | 2.6 | 260 | 1244 | 3 | 0.241 | 1 | 14.7 | |
| MA103 B | icx_16 | 0.02 | N | 1 | 3 | 2 | 2 | 2.1 | 153 | 1244 | 0 | 0.000 | 0 | 7.2 | 120 |
| | icx_21 | 0.065 | N | 4.5 | 5 | 5 | 3 | 3.7 | 153 | 1244 | 0 | 0.000 | 0 | 20.9 | |
| | icx_24 | 0.07 | N | 4.5 | 4 | 5 | 2 | 3 | 153 | 1244 | 0 | 0.000 | 0 | 19.5 | |
| | icx_32 | 0.06 | Y | 5 | 4 | 4 | 3 | 3.2 | 153 | 1244 | 0 | 0.000 | 0 | 21.4 | |
| | icx_33 | 0.05 | N | 3.5 | 5 | 5 | 1 | 2.9 | 153 | 1244 | 0 | 0.000 | 0 | 16.3 | |
| | icx_35 | 0.095 | Y | 5 | 4 | 5 | 5 | 4.2 | 153 | 1244 | 0 | 0.000 | 0 | 23.4 | |
| | icx_37 | 0.035 | N | 2.5 | 4 | 2 | 1 | 2 | 153 | 1244 | 0 | 0.000 | 0 | 11.5 | |

According to the scoring results in the above table, it may be seen that, the adjuvants MA103A and MA103B have better compatibility with the 7 peptides, so that the 7 peptides and MA103A or MA103B have the potential to be compatible with the vaccine formulation.

It is to be noted that, for ease of simple description, the foregoing method embodiments are all expressed as a series of action combinations, but those skilled in the art should know that the present invention is not limited by the described action sequence, as according to the present invention, some steps may be performed in other sequences or simultaneously. Then, those skilled in the art should also know that the embodiments described in the specification are all preferred embodiments, and the actions involved are not necessarily required by the present invention.

Through the description of the above implementations, those skilled in the art may clearly understand that this application can be implemented by means of software and necessary hardware devices such as detection instruments. Based on this understanding, a data processing portion in the above technical solution of this application can be embodied in the form of a software product. The computer software product can be stored in a storage medium, such as ROM/RAM, a magnetic disk, an optical disk, and the like, and includes a plurality of instructions to cause a computer device (which may be a personal computer, a server, or a network device, or the like) to perform the method described in various embodiments of this application or some parts of the embodiments.

Third portion instruments and devices capable of performing the above method for screening an antigen epitope polypeptide

### Embodiment I

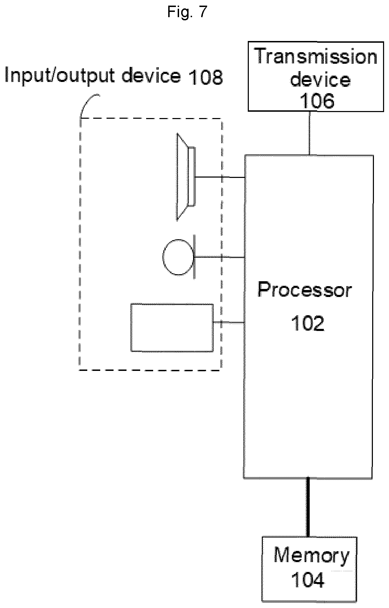

The method provided in the foregoing embodiments of this application may be performed in a terminal, a computer terminal, or a similar computing apparatus. By being operated on the terminal as an example, Fig. 7 is a block diagram of a hardware structure of a terminal of a method for screening an antigen epitope polypeptide according to an embodiment of the present invention. As shown in Fig. 7, the terminal may include one or more (only one is shown in Fig. 7) processors 102 (the processor 102 may include, but is not limited to, a processing apparatus such as a microprocessor MCU or a programmable logic device FPGA) and a memory 104 for storing data. Optionally, the above terminal may further include a transmission device 106 for achieving a communication function and an input/output device 108. Those skilled in the art may understand that the structure shown in Fig. 7 is only a schematic diagram, which does not limit the structure of the above terminal. For example, the terminal may also include more or less components than those shown in Fig. 7, or have a different configuration from that shown in Fig. 7.

The memory 104 may be configured to store a computer program, for example, a software program and a module of application software, such as a computer program corresponding to a method for screening an antigen epitope polypeptide in the embodiments of the present invention. The processor 102 operates the computer program stored in the memory 104, so as to perform various functional applications and data processing, that is, to realize the above method. The memory 104 may include a high-speed random access memory, and may further include a non-volatile memory, such as one or more magnetic disk memory apparatuses, a flash memory device, or other non-volatile solid-state memory devices. In some embodiments, the memory 104 may further include memories remotely disposed relative to the processor 102. The remote memories may be connected to the terminal by using a network. Examples of the above network include, but are not limited to, the Internet, an intranet, a local area network, a mobile communication network, and a combination thereof.

The transmission device 106 is configured to receive or transmit data via a network. The specific example of the above network may include a wireless network provided by a communication provider of the terminal. In an example, the transmission device 106 includes a Network Interface Controller (NIC), and may be connected to other network devices by using a base station, so as to communicate with the Internet. In an example, the transmission device 106 is a Radio Frequency (RF) module, which is configured to communicate with the Internet in a wireless manner.

### Embodiment II

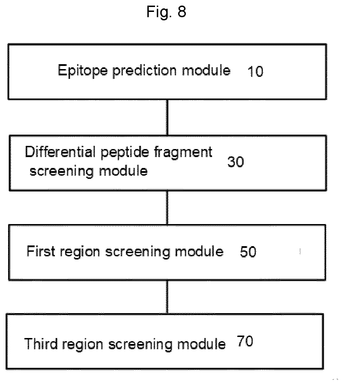

This embodiment provides a device for screening an antigen epitope polypeptide. As shown in Fig. 8, the device includes an epitope prediction module, a differential peptide fragment screening module, a first region screening module, and a third region screening module.

The epitope prediction module 10 is configured to use all proteome sequences of a target coronavirus to perform antigen epitope prediction, to obtain a predicted epitope region.

The differential peptide fragment screening module 30 is configured to use a polypeptide chip technology to screen a polypeptide with a differential response to a positive serum sample infected by the target coronavirus and a control serum sample, and record the polypeptide as a differential peptide fragment.

The first region screening module 50 is configured to compare the differential peptide fragment with all proteome sequences of the target coronavirus to obtain a first conserved motif region.

The third region screening module 70 is configured to screen regions meeting epitope screening conditions from the predicted epitope region and the first conserved motif region to obtain the antigen epitope polypeptide.

The epitope screening conditions include a non-phosphorylation region and/or an extracellular region of the target coronavirus.

Preferably, the epitope prediction module includes: a first candidate epitope screening module, configured to use all proteome sequences of the target coronavirus to perform antigen epitope prediction by means of various methods, and screen epitope with a length of 8 to 20, preferably 10 to 15 amino acids, to obtain candidate prediction epitope; and a second candidate epitope screening module, configured to screen the candidate prediction epitope according to epitope and/or hydrophilicity-hydrophobicity that HLA is able to present in a specific population, to obtain the predicted epitope region.

Preferably, the second candidate epitope screening module includes: a population epitope screening module, configured to screen, from the candidate prediction epitope, the epitope that the HLA is able to present in a Chinese population; and/or a hydrophobicity screening module, configured to remove, from the candidate prediction epitope, the epitope of which hydrophobicity is higher than a first hydrophobic threshold, to obtain the predicted epitope region. Preferably, the epitope of which hydrophobicity is higher than the first hydrophobic threshold refers to epitope that the proportion of hydrophobic amino acids is greater than 45% and a hydrophobicity score is greater than 3.

Preferably, the differential peptide fragment screening module includes a first screening module. The first screening module includes: a sample selection unit, configured to select the positive serum sample infected by the target coronavirus, a negative control serum sample and a control serum sample of another lung disease, where the another lung disease refers to a lung disease caused by infection of a virus other than the target coronavirus; a signal acquisition unit, configured to use a polypeptide chip method to combine the positive serum sample, the negative control serum sample and the control serum sample of the another lung disease with a polypeptide array chip, to obtain signal values responsive to combined peptide fragments; and a differential peptide fragment screening unit, configured to, for each combined peptide fragment, calculate a p value when there is a difference between the signal value of the positive serum sample and the signal value of the negative control serum sample, record the p value as a first p value, and simultaneously, calculate a p value when there is a difference between the signal value of the positive serum sample and the signal value of the control serum sample of the another lung disease, and record the p value as a second p value; and retain all combined peptide fragments of which first p values and second p values simultaneously meet a difference threshold, to obtain the differential peptide fragment. The difference threshold is preferably < 0.05.

Preferably, the differential peptide fragment screening unit includes: a signal conversion sub-unit, configured to perform log10 conversion on the signal value of the combined peptide fragment; and a differential peptide fragment screening sub-unit, configured to use a conversed log value as a feature, by means of a single-tail T test, calculate the p value of each feature when there is a difference between the positive serum sample and the negative control serum sample, and perform multiple hypothesis test correction on the p value to obtain the first p value; simultaneously calculate the p value of the corresponding feature when there is a difference between the positive serum sample and the control serum sample of the another lung disease, perform multiple hypothesis test correction on the p value, and record the p value as the second p value; and screen all combined peptide fragments of which first p values are less than the difference threshold and second p values are less than the difference threshold simultaneously, to obtain the differential peptide fragment.

Preferably, the first region screening module includes: a conserved site screening module, configured to use a single amino acid as a unit, calculate a distribution of p1 values where the signal value of the combined peptide fragment covering the amino acid and matching the amino acid differs between the positive serum sample and the negative control serum sample, simultaneously calculate a distribution of p2 values where the signal value of the combined peptide fragment covering the amino acid and not matching the amino acid differs between the positive serum sample and the negative control serum sample, and record the amino acid that the distribution of p1 values is remarkably lower than the distribution of p2 values as a first conserved site; and a first conserved motif screening module, configured to compare the differential peptide fragment with all proteome sequences of the target coronavirus, and select, from matching regions, a region that has the first conserved site and has hydrophobicity lower than a second hydrophobic threshold, to obtain a first conserved motif region. Preferably, the region of which hydrophobicity is lower than the second hydrophobic threshold refers to a region that the proportion of the hydrophobic amino acids is less than or equal to 45% and the hydrophobicity score is less than or equal to 3. Preferably, the differential peptide fragment is a differential peptide fragment that is able to completely match all proteome sequences of the target coronavirus.

Preferably, the screening device further includes a second region screening module. Preferably, the second region screening module includes: a comparison module, configured to compare the differential peptide fragment with a protein sequence of a coronavirus family; and a second conserved motif screening module, configured to select, from the matching regions, a region of which amino acid site meets the following region screening condition as the second conserved motif region. In all of the differential peptide fragments covering the amino acids, the ratio of the differential peptide fragments matching the amino acids meets a matching ratio threshold.

Preferably, the matching ratio threshold is greater than or equal to 75%.

Preferably, the epitope screening condition in the third region screening module 50 includes at least one of the following: (a) overlapping with the second conserved motif region; (b) a comparison score with a human proteome sequence being lower than a comparison threshold; and (c) meeting a plurality of the following performance indexes: 1) the covering number of the differential peptide fragment being ≥3; 2) hydrophilicity meeting a hydrophilic threshold; and 3) an accessibility score, a Beta turn and a multi-alignment score being all in the top 100. That the comparison score is lower than the comparison threshold means that a/b ≤ 0.8, where a is a matching score that a sequence of a region to be screened is compared with the human proteome sequence, and b is a matching score that the sequence of the region to be screened is compared with all proteome sequences of the target coronavirus.

Preferably, the third region screening module includes: a merging module, configured to merge the predicted epitope region and the first conserved motif region according to one of the following merging conditions: 1) there is an inclusion relation between the two regions; and 2) the two regions are predicted as antigen epitope regions by at least two different methods, to obtain a first candidate epitope region; an overlap screening module, configured to screen a region overlapping with the second conserved motif region from the first candidate epitope region as a second candidate epitope region; a comparison screening module, configured to screen, from the second candidate epitope region, a region of which comparison score with the human proteome sequence is lower than a first threshold, as a third candidate epitope region; a non-phosphorylation and extracellular region screening module, configured to screen and retain the non-phosphorylation region and/or the extracellular region in the proteome sequence of the target coronavirus from the third candidate epitope region, as a fourth candidate epitope region; and a comprehensive sorting module, configured to comprehensively sort the fourth candidate epitope region according to accessibility, the beta turn, the hydrophilicity, the covering number of the differential peptide fragments and a multi-alignment result, and then perform optimal selection, to obtain the antigen epitope polypeptide of the target coronavirus.

Preferably, the device further includes: a mutation removing module, configured to remove a region including mutations from regions optimally selected by the comprehensive sorting module, to obtain the antigen epitope polypeptide of the target coronavirus.

Preferably, the target coronavirus is SARS-CoV-2.

### Embodiment III

This embodiment provides a storage medium. The storage medium includes a stored program. When the program is operated, a device where the storage medium is located is controlled to execute the method for screening an antigen epitope polypeptide described in any one of the above.

### Embodiment IV

This embodiment provides a processor. The processor is configured to operate a program. When the program is operated, the method for screening an antigen epitope polypeptide described in any one of the above is executed.

Each embodiment in this specification is described in a progressive manner, and reference may be made to each other for the same and similar parts among the various embodiments, and each embodiment focuses on the differences from other embodiments. In particular, for the system embodiments, since the system embodiments are basically similar to the method embodiments, the description is relatively simple, and for related parts, refer to the partial descriptions of the method embodiments.

This application may be used in numerous general purpose or special computing system environments or configurations, for example, personal computers, server computers, handheld devices or portable devices, tablet devices, multiprocessor systems, microprocessor-based systems, set top boxes, programmable consumer electronic devices, network PCs, small computers, large computers, distributed computing environments including any of the above systems or devices, and the like.

It is apparent that those skilled in the art should understand that part of the above mentioned modules or steps of this application may be implemented by a general computing device, and may also be gathered together on a single computing device or distributed in network composed of multiple computing devices. Optionally, the above mentioned modules or steps of this application may be implemented with program codes executable by the computing device, so that may be stored in a storage device for execution by the computing device, or can be fabricated into individual integrated circuit modules respectively, or multiple modules or steps thereof are fabricated into a single integrated circuit module for implementation. In this way, this application is not limited to any specific combination of hardware and software.

It is to be noted that terms "first", "second", "third" and the like in the description, claims and the above mentioned drawings of this application are used for distinguishing similar objects rather than describing a specific sequence or a precedence order. It should be understood that the data used in such a way may be exchanged where appropriate, in order that the embodiments of this application described here can be implemented. In addition, terms "include" and "have" and any variations thereof are intended to cover non-exclusive inclusions. For example, it is not limited for processes, methods, systems, products or devices containing a series of steps or units to clearly list those steps or units, and other steps or units which are not clearly listed or are inherent to these processes, methods, products or devices may be included instead.

The above are only the preferred embodiments of this application and are not intended to limit this application. For those skilled in the art, this application may have various modifications and variations. Any modifications, equivalent replacements, improvements and the like made within the spirit and principle of this application all fall within the scope of protection of the present invention.

### Industrial Applicability

Through the technical method of this application, the present invention has at least the following beneficial effects.

Through the application of the technical solution of the present invention, by innovatively combining the polypeptide chip technology, a batch of polypeptide specifically related to coronavirus infection (especially SARS-Cov-2 virus infection). The polypeptide can be used to prepare related detection reagents such as antigens, antibodies and kits, as well as related vaccine products such as polypeptide vaccines, nucleic acid vaccines and protein recombinant vaccines. Therefore, a more powerful tool can be provided for the prevention and control of the infection and prevalence of such viruses.

## Claims

1. A method for screening an antigen epitope polypeptide, comprising:
predicting one or more antigen epitopes with all proteome sequences of a target coronavirus, to obtain a predicted epitope region;
screening a polypeptide with a differential response to a positive serum sample infected by the target coronavirus and a control serum sample with a polypeptide chip technology, and recording the polypeptide as a differential peptide fragment;
aligning the differential peptide fragment with all proteome sequences of the target coronavirus to obtain a first conserved motif region; and
screening one or more regions meeting an epitope screening condition from the predicted epitope region and the first conserved motif region to obtain the antigen epitope polypeptide, wherein the epitope screening condition comprise a non-phosphorylation region and/or an extracellular region of the target coronavirus.

2. The screening method according to claim 1, wherein predicting one or more antigen epitopes with all proteome sequences of a target coronavirus, to obtain a predicted epitope region comprises:
predicting one or more antigen epitopes with all proteome sequences of the target coronavirus by means of various methods, and screening an epitope with a length of 8 to 20, preferably 10 to 15 amino acids, to obtain a candidate prediction epitope; and
screening the candidate prediction epitope according to hydrophilicity, hydrophobicity and/or epitopes that can be presented by HLA in a specific population, to obtain the predicted epitope region,
preferably, screening, from the candidate prediction epitope, the epitope that is presented by the HLA in a Chinese population, and/or removing, from the candidate prediction epitope, the epitope of which the hydrophobicity is higher than a first hydrophobic threshold, to obtain the predicted epitope region, and
preferably, the epitope of which the hydrophobicity is higher than the first hydrophobic threshold refers to an epitope that the proportion of hydrophobic amino acids is greater than 45% and a hydrophobicity score is greater than 3.

3. The screening method according to claim 1, wherein screening a polypeptide with a differential response to a positive serum sample infected by the target coronavirus and a control serum sample with a polypeptide chip technology, and recording the polypeptide as a differential peptide fragment comprises:
selecting the positive serum sample infected by the target coronavirus, a negative control serum sample and a control serum sample with another lung disease, wherein the another lung disease refers to a lung disease caused by infection of a virus other than the target coronavirus;
combining the positive serum sample, the negative control serum sample and the control serum sample of the another lung disease with a polypeptide array chip with a method of the polypeptide chip technology, to obtain a signal value responsive to a combined peptide fragment;
for each the combined peptide fragment, calculating a p value when there is a difference between the signal value of the positive serum sample and the signal value of the negative control serum sample, recording the p value as a first p value, and simultaneously, calculating a p value when there is a difference between the signal value of the positive serum sample and the signal value of the control serum sample of the another lung disease, and recording the p value as a second p value; and
retaining all combined peptide fragments of which the first p values and the second p values simultaneously meet a difference threshold, to obtain the differential peptide fragment, wherein the difference threshold is preferably < 0.05.

4. The screening method according to claim 3, wherein a log10 conversion is performed on the signal value of the combined peptide fragment, a conversed log value is used as a feature, and by means of a single-tail T test,
calculating a p value of each feature when there is a difference between the positive serum sample and the negative control serum sample, and performing a multiple hypothesis test correction on the p value to obtain the first p value;
simultaneously calculating a p value of the corresponding feature when there is a difference between the positive serum sample and the control serum sample of the another lung disease, and performing a multiple hypothesis test correction on the p value, and the p value is recorded as the second p value; and
screening all combined peptide fragments of which the first p values are less than the difference threshold and the second p values are less than the difference threshold simultaneously, to obtain the differential peptide fragment.

5. The screening method according to claim 3, wherein aligning the differential peptide fragment with all proteome sequences of the target coronavirus to obtain a first conserved motif region comprises:
using a single amino acid as a unit, calculating a distribution of p1 values where the signal value, of the combined peptide fragment covering the amino acid and matching the amino acid, differs between the positive serum sample and the negative control serum sample and the control serum sample of the another lung disease, and simultaneously calculating a distribution of p2 values where the signal value, of the combined peptide fragment covering the amino acid and not matching the amino acid, differs between the positive serum sample and the negative control serum sample and the control serum sample of the another lung disease, wherein the amino acid that the distribution of p1 values is remarkably lower than the distribution of p2 values is a first conserved site; and
aligning the differential peptide fragment with all proteome sequences of the target coronavirus, and selecting, from matching regions, a region that has the first conserved site and has the hydrophobicity lower than a second hydrophobic threshold, to obtain the first conserved motif region,
preferably, the region of which the hydrophobicity is lower than the second hydrophobic threshold refers to a region that the proportion of the hydrophobic amino acids is less than or equal to 45% and the hydrophobicity score is less than or equal to 3, and
preferably, the differential peptide fragment is a differential peptide fragment that is able to completely match all proteome sequences of the target coronavirus.

6. The screening method according to claim 3, wherein before screening one or more regions meeting the epitope screening condition from the predicted epitope region and the first conserved motif region, the screening method further comprises: aligning the differential peptide fragment with a protein sequence of a coronavirus family to obtain a second conserved motif region; and
preferably, aligning the differential peptide fragment with a protein sequence of a coronavirus family to obtain a second conserved motif region comprises:
aligning the differential peptide fragment with the protein sequence of the coronavirus family, and selecting, from the matching regions, a region of which each amino acid site meets the following region screening condition as a second conserved motif region,
in all of the differential peptide fragments covering the amino acids, the ratio of the differential peptide fragments matching the amino acids meets a matching ratio threshold; and
preferably, the matching ratio threshold is greater than or equal to 75%.

7. The screening method according to claim 6, wherein the epitope screening condition comprises at least one of the following:
(a) overlapping with the second conserved motif region;
(b) an alignment score with a human proteome sequence being lower than a alignment threshold; and
(c) meeting a plurality of the following performance indexes: 1) the covering number of the differential peptide fragment being ≥3; 2) the hydrophilicity being within a hydrophilic threshold range; and 3) an accessibility score, a Beta turn and a multi-alignment score being all in the top 100, wherein
that the alignment score is lower than the alignment threshold means that a/b ≤ 0.8, wherein the a is a matching score that a sequence of a region to be screened is aligned with the human proteome sequence, and the b is a matching score that the sequence of the region to be screened is aligned with all proteome sequences of the target coronavirus; and
preferably, the screening regions meeting epitope screening conditions from the predicted epitope region and the first conserved motif region to obtain the antigen epitope polypeptide comprises:
merging the predicted epitope region and the first conserved motif region according to one of the following merging conditions: 1) there is an inclusion relation between the two regions; and 2) the two regions are predicted as antigen epitope regions by at least two different methods, to obtain a first candidate epitope region;
screening a region overlapping with the second conserved motif region from the first candidate epitope region as a second candidate epitope region;
screening, from the second candidate epitope region, a region of which the alignment score with the human proteome sequence is lower than the alignment threshold, as a third candidate epitope region;
screening and retaining the non-phosphorylation region and/or the extracellular region in the proteome sequence of the target coronavirus from the third candidate epitope region, as a fourth candidate epitope region;
comprehensively sorting the fourth candidate epitope region according to accessibility, the beta turn, the hydrophilicity, the covering number of the differential peptide fragments and a multi-alignment result, and then performing optimal selection, to obtain the antigen epitope polypeptide of the target coronavirus; and
more preferably, after the optimal selection is performed, the screening method further comprises removing a region comprising mutations; and
preferably, the target coronavirus is SARS-CoV-2.

8. A device for screening an antigen epitope polypeptide, comprising:
an epitope prediction module, configured to predict one or more epitopes with all proteome sequences of a target coronavirus, to obtain a predicted epitope region;
a differential peptide fragment screening module, configured to screen a polypeptide with a differential response to a positive serum sample infected by the target coronavirus and a control serum sample with a polypeptide chip technology, and record the polypeptide as a differential peptide fragment;
a first region screening module, configured to align the differential peptide fragment with all proteome sequences of the target coronavirus to obtain a first conserved motif region; and
a third region screening module, configured to screen one or more regions meeting an epitope screening condition from the predicted epitope region and the first conserved motif region to obtain the antigen epitope polypeptide,
wherein the epitope screening condition comprise a non-phosphorylation region and/or an extracellular region of the target coronavirus.

9. The screening device according to claim 8, wherein the epitope prediction module comprises:
a first candidate epitope screening module, configured to predict one or more antigen epitopes with all proteome sequences of the target coronavirus by means of various methods, and
screen an epitope with a length of 8 to 20, preferably 10 to 15 amino acids, to obtain a candidate prediction epitope; and
a second candidate epitope screening module, configured to screen the candidate prediction epitope according to hydrophilicity, hydrophobicity and/or epitopes that can be presented by HLA in a specific population, to obtain the predicted epitope region.

10. The screening device according to claim 9, wherein the second candidate epitope screening module comprises:
a population epitope screening module, configured to screen, from the candidate prediction epitope, the epitope that is presented by the HLA in a Chinese population; and/or
a hydrophobicity screening module, configured to remove, from the candidate prediction epitope, the epitope of which the hydrophobicity is higher than a first hydrophobic threshold, to obtain the predicted epitope region, and
preferably, the epitope of which the hydrophobicity is higher than the first hydrophobic threshold refers to an epitope that the proportion of hydrophobic amino acids is greater than 45% and a hydrophobicity score is greater than 3.

11. The screening device according to claim 8, wherein the differential peptide fragment screening module comprises a first screening module; and the first screening module comprises:
a sample selection unit, configured to select the positive serum sample infected by the target coronavirus, a negative control serum sample and a control serum sample of another lung disease, wherein the another lung disease refers to a lung disease caused by infection of a virus other than the target coronavirus;
a signal acquisition unit, configured to combine the positive serum sample, the negative control serum sample and the control serum sample of the another lung disease with a polypeptide array chip with a method of the polypeptide chip technology, to obtain signal values responsive to combined peptide fragments;
a differential peptide fragment screening unit, configured to, for each the combined peptide fragment, calculate a p value when there is a difference between the signal value of the positive serum sample and the signal value of the negative control serum sample, record the p value as a first p value, and simultaneously, calculate a p value when there is a difference between the signal value of the positive serum sample and the signal value of the control serum sample of the another lung disease, and record the p value as a second p value; and retain all combined peptide fragments of which the first p values and the second p values simultaneously meet a difference threshold, to obtain the differential peptide fragment,
preferably, the difference threshold is preferably < 0.05.

12. The screening device according to claim 11, wherein the differential peptide fragment screening unit comprises:
a signal conversion sub-unit, configured to perform a Iog10 conversion on the signal value of the combined peptide fragment; and
a differential peptide fragment screening sub-unit, configured to use a conversed log value as a feature, by means of a single-tail T test, calculate the p value of each feature when there is a difference between the positive serum sample and the negative control serum sample, and perform a multiple hypothesis test correction on the p value to obtain the first p value; simultaneously calculate the p value of the corresponding feature when there is a difference between the positive serum sample and the control serum sample of the another lung disease, perform a multiple hypothesis test correction on the p value, and record the p value as the second p value; and screen all combined peptide fragments of which the first p values are less than the difference threshold and the second p values are less than the difference threshold simultaneously, to obtain the differential peptide fragment.

13. The screening device according to claim 11, wherein the first region screening module comprises:
a conserved site screening module, configured to use a single amino acid as a unit, calculate a distribution of p1 values where the signal value of the combined peptide fragment covering the amino acid and matching the amino acid differs between the positive serum sample and the negative control serum sample, simultaneously calculate a distribution of p2 values where the signal value of the combined peptide fragment covering the amino acid and not matching the amino acid differs between the positive serum sample and the negative control serum sample, and record the amino acid that the distribution of p1 values is remarkably lower than the distribution of p2 values as a first conserved site; and
a first conserved motif screening module, configured to align the differential peptide fragment with all proteome sequences of the target coronavirus, and select, from matching regions, a region that has the first conserved site and has the hydrophobicity lower than a second hydrophobic threshold, to obtain the first conserved motif region,
preferably, the region of which the hydrophobicity is lower than the second hydrophobic threshold refers to a region that the proportion of the hydrophobic amino acids is less than or equal to 45% and the hydrophobicity score is less than or equal to 3, and
preferably, the differential peptide fragment is a differential peptide fragment that is able to completely match all proteome sequences of the target coronavirus.

14. The screening device according to claim 8, further comprising a second region screening module; and the second region screening module comprises:
an alignment module, configured to align the differential peptide fragment with a protein sequence of a coronavirus family; and
a second conserved motif screening module, configured to select, from the matching regions, a region of which each amino acid site meets the following region screening condition as a second conserved motif region, wherein, in all of the differential peptide fragments covering the amino acids, the ratio of the differential peptide fragments matching the amino acids meets a matching ratio threshold; and
preferably, the matching ratio threshold is greater than or equal to 75%.

15. The screening device according to claim 14, wherein the epitope screening condition in the third region screening module comprises at least one of the following: (i) overlapping with the second conserved motif region; (ii) an alignment score with a human proteome sequence being lower than an alignment threshold; and; and (iii) meeting a plurality of the following performance indexes: 1) the covering number of the differential peptide fragment being ≥3; 2) the hydrophilicity being within a hydrophilic threshold range; and 3) an accessibility score, a Beta turn and a multi-alignment score being all in the top 100, wherein, that the alignment score is lower than the alignment threshold means that a/b ≤ 0.8, wherein the a is a matching score that a sequence of a region to be screened is compared with the human proteome sequence, and the b is a matching score that the sequence of the region to be screened is aligned with all proteome sequences of the target coronavirus.

16. The screening device according to claim 15, wherein the third region screening module comprises:
a merging module, configured to merge the predicted epitope region and the first conserved motif region according to one of the following merging conditions: 1) there is an inclusion relation between the two regions; and 2) the two regions are predicted as antigen epitope regions by at least two different methods, to obtain a first candidate epitope region;
an overlap screening module, configured to screen a region overlapping with the second conserved motif region from the first candidate epitope region as a second candidate epitope region;
an alignment screening module, configured to screen, from the second candidate epitope region, a region of which the alignment score with the human proteome sequence is lower than a first threshold, as a third candidate epitope region;
a non-phosphorylation and extracellular region screening module, configured to screen and
retain the non-phosphorylation region and/or the extracellular region in the proteome sequence of the target coronavirus from the third candidate epitope region, as a fourth candidate epitope region; and
a comprehensive sorting module, configured to comprehensively sort the fourth candidate epitope region according to accessibility, the beta turn, the hydrophilicity, the covering number of the differential peptide fragments and a multi-alignment result, and then perform optimal selection, to obtain the antigen epitope polypeptide of the target coronavirus.

17. The screening device according to claim 16, further comprising: a mutation removing module, configured to remove a region comprising mutations from regions optimally selected by the comprehensive sorting module, to obtain the antigen epitope polypeptide of the target coronavirus,
preferably, the target coronavirus is SARS-CoV-2.

18. A storage medium, comprising a stored program, wherein, when the program is operated, a device where the storage medium is located is controlled to execute the method for screening an antigen epitope polypeptide according to any of claims 1 to 7.

19. A processor, configured to operate a program, wherein the method for screening an antigen epitope polypeptide according to any of claims 1 to 7 is executed when the program is operated.
